# EUROPEAN PATENT APPLICATION

(11) **EP 3 366 345 A1**
(43) Date of publication of application: **29.08.2018**
(21) Application number: 18156775.1
(22) Date of filing: 14.02.2018
(51) Int. Cl.: A61M 37/00

(54) **MANUFACTURING METHOD OF SHEET HAVING NEEDLE-LIKE PROTRUSIONS**

(30) Priority: 28.02.2017 JP 2017036752
(71) Applicant: FUJIFILM Corporation, Tokyo 106-8620 (JP)
(72) Inventor: OKANO, Keio, Kanagawa, 258-8577 (JP); KATAGIRI, Yoshinobu, Kanagawa, 258-8577 (JP); WAKAMATSU, Satoshi, Kanagawa, 258-8577 (JP); TAKANO, Ikuo, Kanagawa, 258-8577 (JP)
(74) Representative: Klunker IP Patentanwälte PartG mbB

(57) **Abstract**

Provided is a manufacturing method of a sheet having needle-like protrusions capable of individually manufacturing sheets on a mold. The manufacturing method of a sheet having needle-like protrusions includes, for a mold provided with needle-like recesses and an annular groove provided around a region where the needle-like recesses are formed, a first polymer solution supplying step of supplying a first polymer solution, which is to become a first layer, to the needle-like recesses; a second polymer solution supplying step of filling the needle-like recesses and the groove with a second polymer solution by supplying the second polymer solution, which is to become a second layer, to a surface of the mold; a drying step of forming a laminate of the first layer and the second layer by drying the first polymer solution and the second polymer solution; and a peeling step of peeling the laminate away from the mold.

## Description

### BACKGROUND OF THE INVENTION

### 1. Field of the Invention

The present invention relates to a manufacturing method of a sheet having needle-like protrusions, and particularly to a method of manufacturing a sheet having needle-like protrusions by shape transfer using a mold in which needle-like recesses are formed.

### 2. Description of the Related Art

As a sheet having needle-like protrusions, for example, there is a percutaneous absorption sheet in which needle-like protrusions (also referred to as microneedles) containing a drug and having a high aspect ratio are formed. By inserting the microneedles into the skin, medicine or the like is administered from the percutaneous absorption sheet.

As a method of manufacturing a percutaneous absorption sheet, there is known a method in which a polymer solution or the like is poured to a mold in which needle-like recesses having an inverted shape of needle-like protrusions are formed and shape transfer is performed.

For example, in JP2016-168325A, a method of manufacturing a percutaneous absorption sheet by using a mold provided with a step portion around a region where needle-like recesses are formed, supplying a polymer layer forming liquid, and drying the resultant is described. In JP2016-106676A, a method of manufacturing a needle-like body by supplying a needle-like body forming aqueous solution to an intaglio plate, and drying and peeling the resultant is described.

### SUMMARY OF THE INVENTION

As described in JP2016-106676A, by collectively molding a plurality of microneedles through a single molding process and thereafter cutting the resultant, a large number of microneedles can be molded. However, there is concern that cost may be necessary for a sheet portion between patches to be cut, or dust may be generated by the cutting, leading to the entrainment of dust.

In order to form sheets individually, a polymer solution needs to be held in each of the sheets on a mold (stamper) having needle-like recesses. In the method described in JP2016-168325A, the step portion which is higher or lower than the region where the needle-like recesses are formed around the region where the needle-like recesses are formed. Therefore, the polymer solution can be prevented from spreading by the high step portion, and the polymer solution is fixed by the low step portion, thereby stabilizing the shape of a sheet portion. However, in the case where the step portion higher than the region where the needle-like recesses are formed is provided, there is a problem that the step portion becomes an obstacle during filling of the needle-like recesses with the liquid. Particularly, in the percutaneous absorption sheet containing a drug, the needle-like recesses are first filled with a liquid containing the drug in order to cause the distal end of the needle-like recess to contain the drug. In this case, since the drug is expensive, it is desirable to fill only the needle-like recesses. However, due to the step portion, it is difficult to fill only the needle-like recesses. In addition, in the case where the step portion lower than the region where the needle-like recesses are formed, the liquid containing the drug is accumulated in the step portion, and there is a problem that costs increase.

As another method, it is considered that a liquid is held for each patch in a separate sheet and is bonded to a mold. However, in this method, there is a problem that the polymer liquid needs to be dried after being bonded, and the drying takes time. In order to reduce the drying time, it is considered that the separate sheet is formed of a semi-permeable sheet. However, this results in problems with the adhesiveness of the sheet, the cost to the sheet member, and the cost to the bonding step, which is not desirable.

The present invention has been made taking the above circumstances into consideration, and an object thereof is to provide a manufacturing method of a sheet having needle-like protrusions capable of individually manufacturing sheets having needle-like protrusions by pinning a liquid on a mold to be held and drying the liquid.

In order to achieve the object, the present invention provides a manufacturing method of a sheet having needle-like protrusions comprising: for a mold provided with needle-like recesses and an annular groove provided around a region where the needle-like recesses are formed, a first polymer solution supplying step of supplying a first polymer solution, which is to become a first layer, to the needle-like recesses; a second polymer solution supplying step of filling the needle-like recesses and the groove with a second polymer solution by supplying the second polymer solution, which is to become a second layer, to a surface of the mold; a drying step of forming a laminate of the first layer and the second layer by drying the first polymer solution and the second polymer solution; and a peeling step of peeling the laminate away from the mold.

According to the present invention, since the annular groove is provided around the region where the needle-like recesses are formed and the groove is filled with the second polymer solution, in a case where the second polymer solution is repelled by the mold or the second polymer solution is dried in the drying step, the contraction of the second polymer solution can be stopped by the groove. By filling the groove with the second polymer solution, the second polymer solution and the mold can be brought into surface contact with each other, and the contraction of the second polymer solution can be easily stopped by the groove. In a case where the groove is not filled with the second polymer solution, the corner of the groove and the second polymer solution are brought into point or line contact with each other, and the second polymer solution is less likely to be fixed in the groove.

By fixing the second polymer solution in the groove, the sheet can be formed into the size of the annular groove. Therefore, without a step of cutting the sheet, sheets having a constant size can be formed on the mold.

According to another aspect of the present invention, it is preferable that after the second polymer solution supplying step, the second polymer solution is pinned in the groove.

According to the aspect, by pinning the second polymer solution in the groove, a sheet having the size of the groove can be reliably formed.

In the present invention, "pinning" means that in a case where a polymer solution is supplied onto a mold and in a case where the polymer solution is viewed in a two-dimensional section in a mold thickness direction and a radial direction of a region where needle-like recesses are formed during drying and solidification of the polymer solution, the three phase contact lines of the polymer solution, the mold surface, and the air do not move.

According to another aspect of the present invention, it is preferable that a width of the groove is 2 mm or less.

According to the aspect, by causing the width of the groove to be in the above range, the amount of the second polymer solution filling the groove can be reduced. In addition, in the first polymer solution supplying step, the groove can be prevented from being filled with the first polymer solution. In a case where the sheet having needle-like protrusions is a percutaneous absorption sheet, since the first polymer solution contains the drug, the amount of the consumed first polymer solution can be suppressed, thereby preventing an increase in costs.

According to another aspect of the present invention, it is preferable that the groove consists of a plurality of annular grooves and further has one or more annular grooves around a single annular groove.

According to the aspect, since the groove consists of the plurality of annular grooves, in a case where the second polymer solution cannot be fixed in the outermost groove farthest from the region where needle-like protrusions are formed, the second polymer solution is fixed in the inner groove, and the sheet in a constant size standard can be reliably formed.

According to another aspect of the present invention, it is preferable that the plurality of annular grooves are concentric grooves having different radii.

According to the aspect, since the second polymer solution supplied onto the mold contracts in a direction toward the center, the second polymer solution can be easily fixed in the groove by using the concentric grooves.

According to another aspect of the present invention, it is preferable that an angle of a corner formed by a wall surface of the groove on a side of a region where the needle-like recesses are formed and the surface of the mold is an obtuse angle, and in the second polymer solution supplying step, the second polymer solution is supplied from the needle-like recesses side to fill the groove.

According to the aspect, regarding the shape of the groove, by causing the corner of the wall surface on the region side where the needle-like recesses are formed to be at an obtuse angle and supplying the second polymer solution from the needle-like recess side, the groove can be easily filled with the second polymer solution. By filling the groove with the second polymer solution, the second polymer solution can be fixed in the groove. In addition, by causing the wall surface on the needle-like recess side to be at an obtuse angle and supplying the second polymer solution from the wall surface side at the obtuse angle, the entrainment of bubbles (air) into the groove can be prevented. Therefore, at the position where the second polymer solution is fixed, the deviation of the fixing of the second polymer solution due to the incorporation of bubbles can be prevented.

According to another aspect of the present invention, it is preferable that an angle of a corner formed by a wall surface of the groove on the opposite side to a region where the needle-like recesses are formed and the surface of the mold is an obtuse angle, and in the second polymer solution supplying step, the second polymer solution is supplied from around the groove to fill the needle-like recesses and the groove.

According to the aspect, regarding the shape of the groove, by causing the corner of the wall surface on the opposite side to the region side where the needle-like recesses are formed to be at an obtuse angle and supplying the second polymer solution from around the groove, the groove can be easily filled with the second polymer solution. In addition, by causing the opposite side to the region where the needle-like recesses are formed to be at an obtuse angle and supplying the second polymer solution from the wall surface side at the obtuse angle, the entrainment of bubbles (air) into the groove can be prevented. Therefore, at the position where the second polymer solution is fixed, the deviation of the fixing of the second polymer solution due to the incorporation of bubbles can be prevented.

According to another aspect of the present invention, it is preferable that the obtuse angle is an angle of more than 90° and equal to or less than 135°.

According to the aspect, by causing the angle of the obtuse angle to be in the above range, filling of the groove with the second polymer solution and fixing of the second polymer solution can be made compatible with each other. In a case where the angle is smaller than the above range, it is difficult to fill the groove with the second polymer solution. In a case where the angle is larger than the above range, there is a need to increase the width of the groove to deepen the groove, which is not preferable.

According to another aspect of the present invention, it is preferable that an angle of a corner formed by a wall surface of the groove on the opposite side to a side where the second polymer solution is supplied and the surface of the mold is smaller than an angle of a corner formed by a wall surface on the side where the second polymer solution is supplied and the surface of the mold.

According to the aspect, by setting the angles of the corners formed by the wall surface on the side where the second polymer solution is supplied and the wall surface on the opposite side thereto, and the surface of the mold to cause the angle of the wall surface on the opposite side to the side where the second polymer solution is supplied to be smaller, the second polymer solution can be easily filled from one side, and the second polymer solution can be fixed by the other wall surface of the groove.

According to another aspect of the present invention, it is preferable that the angle of the corner formed by the wall surface of the groove on the opposite side to the side where the second polymer solution is supplied and the surface of the mold is a right angle.

According to the aspect, by causing the angle of the wall surface on the opposite side to the side where the second polymer solution is supplied to be a right angle, the probability that the second polymer solution will be fixed can be improved.

According to another aspect of the present invention, it is preferable that the mold is formed of a gas permeable material, and in the second polymer solution supplying step, the groove is closed with the second polymer solution and thereafter the second polymer solution is suctioned from a side opposite to a surface of the mold in which the needle-like recesses and the groove are formed.

According to the aspect, the groove is closed with the second polymer solution and thereafter the second polymer solution is suctioned from the side opposite to the surface of the mold in which the groove is formed, the air in the groove can be released. Therefore, the groove can be reliably filled with the second polymer solution and the entrainment of bubbles (air) into the groove can be prevented.

According to another aspect of the present invention, it is preferable that the first polymer solution supplying step is performed while bringing a distal end of an applicator that applies the first polymer solution into contact with the mold.

In the present invention, by providing the groove in the surface of the mold and fixing the second polymer solution therein, even in a case where the first polymer solution is supplied while bringing the distal end of the applicator into contact with the surface of the mold, the supply can be performed without an obstacle. In a case where a protrusion is provided and the first polymer solution is supplied while bringing the distal end of the applicator into contact with the mold, the first polymer solution is caught on the step of the protrusion and is wasted, which is not preferable.

According to another aspect of the present invention, it is preferable that a surface tension of the second polymer solution is low.

According to the aspect, by causing the surface tension of the polymer solution to be low, wettability to the surface of the mold can be improved, and the second polymer solution can be easily fixed on the surface of the mold. It is preferable that the surface tension is lower than the surface tension of water (72.7 mN/m).

According to another aspect of the present invention, it is preferable that the first polymer solution includes a drug.

According to the aspect, by causing the first polymer solution to contain the drug, the drug can be contained only the distal end of the needle-like protrusion, and the sheet can be suitably used as a percutaneous absorption sheet.

According to the manufacturing method of a sheet having needle-like protrusions of the present invention, the polymer solution can be fixed in the groove provided in the surface of the mold, and the size of the sheet portion can be determined by the groove. Therefore, sheets can be individually formed and do not require cutting. Therefore, the cost to unnecessary sheet portions to be cut is not required, and the generation of dust due to cutting and the entrainment of the dust can be prevented.

### BRIEF DESCRIPTION OF THE DRAWINGS

Fig. 1 is a perspective view of a percutaneous absorption sheet having a needle-like protrusion.
Fig. 2 is a perspective view of a percutaneous absorption sheet having a needle-like protrusion with another shape.
Fig. 3 is a sectional view of the needle-like protrusions of the percutaneous absorption sheets illustrated in Figs. 1 and 2.
Fig. 4 is a perspective view of a percutaneous absorption sheet having a needle-like protrusion with another shape.
Fig. 5 is a perspective view of a percutaneous absorption sheet having a needle-like protrusion with another shape.
Fig. 6 is a sectional view of the needle-like protrusions of the percutaneous absorption sheets illustrated in Figs. 4 and 5.
Fig. 7 is a process diagram of a manufacturing method of a mold.
Fig. 8 is a process diagram of the manufacturing method of a mold.
Fig. 9 is a process diagram of the manufacturing method of a mold.
Fig. 10 is a plan view of the mold.
Fig. 11 is a partial enlarged view of the mold.
Fig. 12 is a partial enlarged view of a mold.
Fig. 13 is a flowchart of a manufacturing method of a percutaneous absorption sheet.
Fig. 14 is a schematic view illustrating a step of filling needle-like recesses of the mold with a first polymer solution.
Fig. 15 is a schematic view illustrating a step of filling the needle-like recesses of the mold with the first polymer solution.
Fig. 16 is a schematic view illustrating a step of filling the needle-like recesses of the mold with the first polymer solution.
Fig. 17 is a perspective view illustrating the distal end of a nozzle.
Fig. 18 is a perspective view illustrating the distal end of another nozzle.
Fig. 19 is a partial enlarged view of the distal end of the nozzle and the mold during filling.
Fig. 20 is a partial enlarged view of the distal end of the nozzle and the mold during scanning.
Fig. 21 is a schematic configuration diagram of a polymer solution filling apparatus.
Fig. 22 is an explanatory view illustrating the relationship between the liquid pressure in the nozzle and the supply of the first polymer solution.
Fig. 23 is a schematic view illustrating a part of a manufacturing step of a percutaneous absorption sheet.
Fig. 24 is a schematic view illustrating a part of the manufacturing step of a percutaneous absorption sheet.
Fig. 25 is a schematic view illustrating a part of the manufacturing step of a percutaneous absorption sheet.
Fig. 26 is a schematic view illustrating a part of the manufacturing step of a percutaneous absorption sheet.
Fig. 27 is a schematic view illustrating a part of the manufacturing step of a percutaneous absorption sheet.
Fig. 28 is a sectional view of another mold.
Fig. 29 is a plan view of another mold.
Fig. 30 is a view illustrating another embodiment of a second polymer solution supplying step.
Fig. 31 is a sectional view of a mold preferably used in the polymer solution supplying step illustrated in Fig. 30.
Fig. 32 is a view illustrating still another embodiment of the second polymer solution supplying step.
Fig. 33 is sectional view of a mold preferably used in the polymer solution supplying step illustrated in Fig. 32.
Fig. 34 is a plan view and a sectional view of a plate precursor used in examples.

### DESCRIPTION OF THE PREFERRED EMBODIMENTS

Hereinafter, a manufacturing method of a sheet having needle-like protrusions according to the present invention will be described with reference to the attached drawings. In the present specification, "to" is used to include numerical values described before and after "to" as the lower limit and the upper limit.

### (Sheet Having Needle-like Protrusions)

As an example of a sheet having needle-like protrusions manufactured in this embodiment, a percutaneous absorption sheet (microneedle sheet) will be described. The sheet having needle-like protrusions is not limited to the percutaneous absorption sheet, and another sheet having needle-like protrusions can also be used. As another sheet having needle-like protrusions, microarrays and microlens arrays for protein and cell analysis, substrates for heat dissipation and heat absorption, substrates for sound absorption, filters, members for forming microchannels, and the like may be employed.

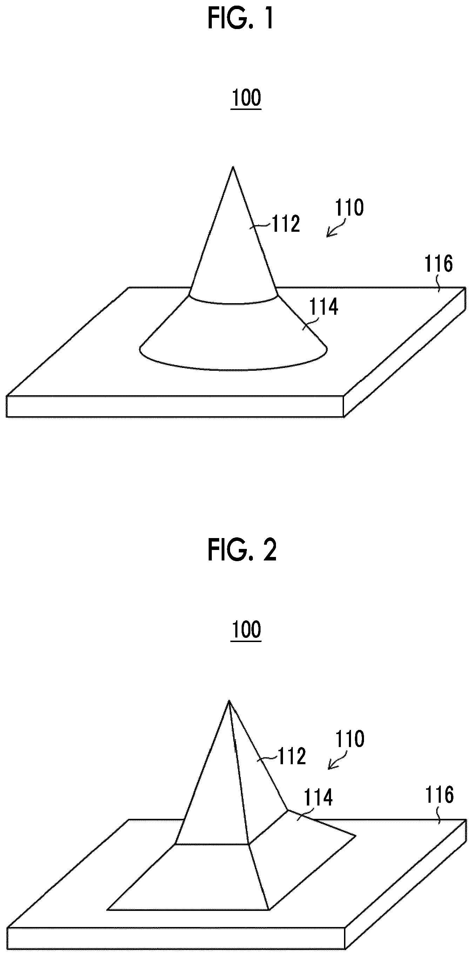

Figs. 1 and 2 illustrate needle-like protrusions 110 (also referred to as microneedles) in partially enlarged views of percutaneous absorption sheets 100.

The percutaneous absorption sheet 100 is attached to the skin such that a drug is supplied to the skin. As illustrated in Fig. 1, the percutaneous absorption sheet 100 includes a tapered needle portion 112, a frustum portion 114 connected to the needle portion 112, a flat plate-shaped sheet portion 116 connected to the frustum portion 114. The needle-like protrusion 110 is constituted by the tapered needle portion 112 and the frustum portion 114.

A plurality of the frustum portions 114 are formed on the surface of the sheet portion 116 (only one frustum portion 114 is illustrated in Fig. 1). An end surface (lower base) having a larger area among the two end surfaces of the frustum portion 114 is connected to the sheet portion 116. An end surface (upper base) having a smaller area among the two end surfaces of the frustum portion 114 is connected to the needle portion 112. That is, the area of the end surface in a direction away from the sheet portion 116 among the two end surfaces of the frustum portion 114 is small. Since the surface of the needle portion 112 having a large area is connected to the end surface of the frustum portion 114 having a smaller area, the needle portion 112 has a shape gradually tapered in a direction away from the frustum portion 114.

In Fig. 1, the frustum portion 114 has a truncated cone shape, and the needle portion 112 has a conical shape. The shape of the distal end of the needle portion 112 can be appropriately changed to a curved surface having a radius of curvature of 0.01 µm or more and 50 µm or less, a flat surface, or the like according to the degree of insertion of the needle portion 112 into the skin.

Fig. 2 illustrates the needle-like protrusion 110 having another shape. In Fig. 2, the frustum portion 114 has a truncated pyramid shape, and the needle portion 112 has a square pyramid shape.

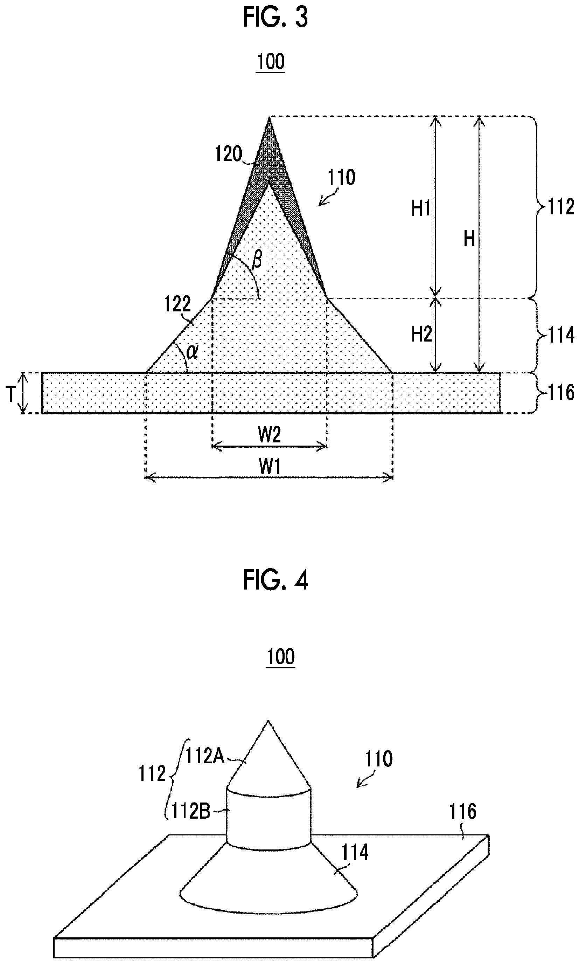

Fig. 3 is a sectional view of the percutaneous absorption sheets 100 illustrated in Figs. 1 and 2. As illustrated in Fig. 3, the percutaneous absorption sheets 100 are formed of a drug layer 120 containing a predetermined amount of a drug and a polymer layer 122. Here, containing a predetermined amount of a drug means containing a drug in an amount that exhibits a medicinal effect as the body surface is punctured. The drug layer 120 containing the drug is formed at the distal end of the needle-like protrusion 110 (the distal end of the needle portion 112). By forming the drug layer 120 at the distal end of the needle-like protrusion 110, the drug can be efficiently delivered into the skin. Hereinafter, "containing a predetermined amount of a drug" is referred to as "containing a drug" as necessary.

The polymer layer 122 is formed in a portion of the needle portion 112 excluding the drug layer 120. The frustum portion 114 is formed of the polymer layer 122. The sheet portion 116 is formed of the polymer layer 122. The distribution of the drug layer 120 and the polymer layer 122 forming the needle portion 112, the frustum portion 114, and the sheet portion 116 can be appropriately set.

The thickness T of the sheet portion 116 is in a range of 10 µm to 2000 µm, and preferably in a range of 10 µm to 1000 µm. The width W1 of the portion (lower base) of the frustum portion 114 that is in contact with the sheet portion 116 is in a range of 100 µm to 1500 µm, and preferably in a range of 100 µm to 1000 µm. The width W2 of the portion (upper base) of the frustum portion 114 that is in contact with the needle portion 112 is in a range of 100 µm to 1500 µm, and preferably in the range of 100 µm to 1000 µm. The width W1 and the width W2 satisfy W1 > W2 in the above numerical value ranges.

The height H of the needle-like protrusion 110 is in a range of 100 µm to 2000 µm, and preferably in the range of 200 µm to 1500 µm. Regarding H1/H2 which is the ratio of the height HI of the needle portion 112 to the height H2 of the frustum portion 114, H1/H2 is in a range of 1 to 10, and preferably in a range of 1.5 to 8. Furthermore, the height H2 of the frustum portion 114 is preferably in a range of 10 µm to 1000 µm.

The angle α between the side surface of the frustum portion 114 and the surface parallel to the surface of the sheet portion 116 is in a range of 10° to 60°, and preferably in a range of 20° to 50°. The angle β between the side surface of the needle portion 112 and the surface parallel to the upper base of the frustum portion 114 is in a range of 45° to 85°, and preferably in a range of 60° to 80°.

It is preferable that the angle β is equal to or more than the angle α. This is because the needle-like protrusion 110 can be easily inserted into the skin.

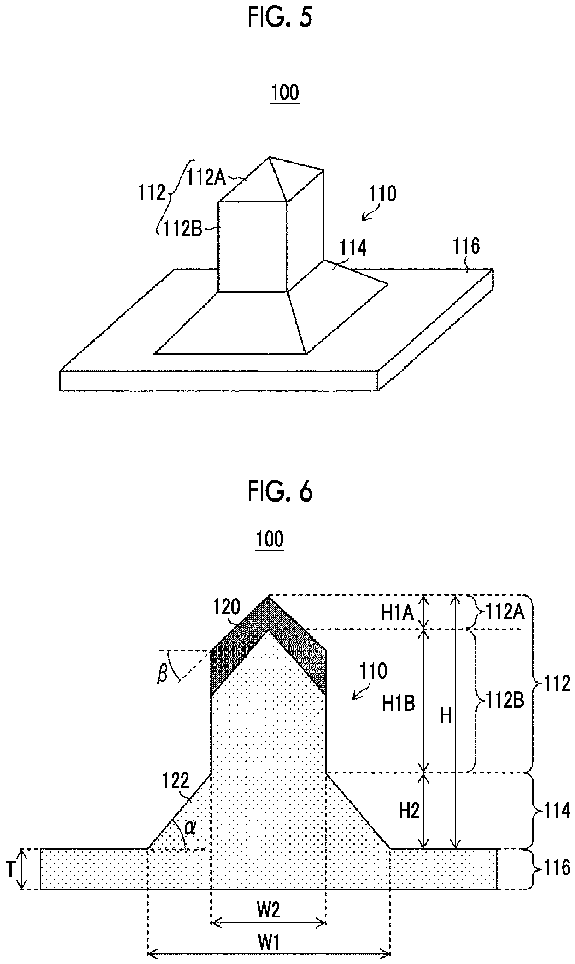

Figs. 4 and 5 illustrate needle-like protrusions 110 having other shapes. Each of the percutaneous absorption sheets 100 illustrated in Figs. 1 and 4 and the percutaneous absorption sheets 100 illustrated in Figs. 2 and 5 are the same in the shape of the frustum portion 114 and are different from each other in the shape of the needle portion 112. Needle portions 112 illustrated in Figs. 4 and 5 have a tapered needle-like portion 112A and a tubular body portion 112B. The bottom surface of the needle-like portion 112A and the end surface of the body portion 112B are connected to each other. An end surface of the body portion 112B which is not connected to the needle-like portion 112A among the end surfaces of the body portion 112B and the upper base of the frustum portion 114 are connected to each other.

The needle-like portion 112A illustrated in Fig. 4 has a conical shape and the body portion 112B has a cylindrical shape. The needle-like portion 112A illustrated in Fig. 5 has a square pyramid shape, and the body portion 112B has a square prism shape.

Since the needle portion 112 has the body portion 112B, the needle portion 112 has a shape having a constant width in the direction away from the frustum portion 114. The needle-like portion 112A of the needle portion 112 has a shape gradually tapered in the direction away from the body portion 112B. The areas of the two opposed end surfaces of the tubular body portion 112B are substantially the same. The needle portion 112 has a tapered shape as a whole. The shape of the distal end of the needle portion 112 can be appropriately changed to a curved surface having a radius of curvature of 0.01 µm or more and 50 µm or less, a flat surface, or the like according to the degree of insertion of the needle portion 112 into the skin.

Fig. 6 is a sectional view of the percutaneous absorption sheets 100 illustrated in Figs. 4 and 5. As illustrated in Fig. 6, the percutaneous absorption sheets 100 are formed of a drug layer 120 containing a drug and a polymer layer 122. The drug layer 120 containing the drug is formed at the distal end of the needle-like protrusion 110 (the distal end of the needle portion 112). By forming the drug layer 120 at the distal end of the needle-like protrusion 110, the drug can be efficiently supplied into the skin.

The polymer layer 122 is formed in a portion of the needle portion 112 excluding the drug layer 120. The frustum portion 114 is formed of the polymer layer 122. A sheet portion 116 is formed of the polymer layer 122. The distribution of the drug layer 120 and the polymer layer 122 forming the needle portion 112, the frustum portion 114, and the sheet portion 116 can be appropriately set.

The thickness T of the sheet portion 116, the width W1 of the lower base of the frustum portion 114, the width W2 of the upper base of the frustum portion 114, the height H of the needle-like protrusion 110, and the height H2 of the frustum portion 114 can be the same as the length of the percutaneous absorption sheet 100 illustrated in Fig. 3.

Regarding H1B/H1A which is the ratio of the height H1A of the needle-like portion 112A to the height H1B of the body portion 112B, H1B/H1A is in a range of 0.1 to 4, and preferably in a range of 0.3 to 2.

The angle α between the side surface of the frustum portion 114 and the surface parallel to the surface of the sheet portion 116 is in a range of 10° to 60°, and preferably in a range of 20° to 50°. The angle β between the side surface of the needle-like portion 112A and the surface parallel to the end surface of the body portion 112B is in a range of 45° to 85°, and preferably in a range of 60° to 80°.

It is preferable that the angle β is equal to or more than the angle α. This is because the needle-like protrusion 110 can be easily inserted into the skin.

In this embodiment, although the percutaneous absorption sheets 100 having the needle portions 112 illustrated in Figs. 1, 2, 4, and 5 are described, the percutaneous absorption sheets 100 are not limited to these shapes.

### (Mold)

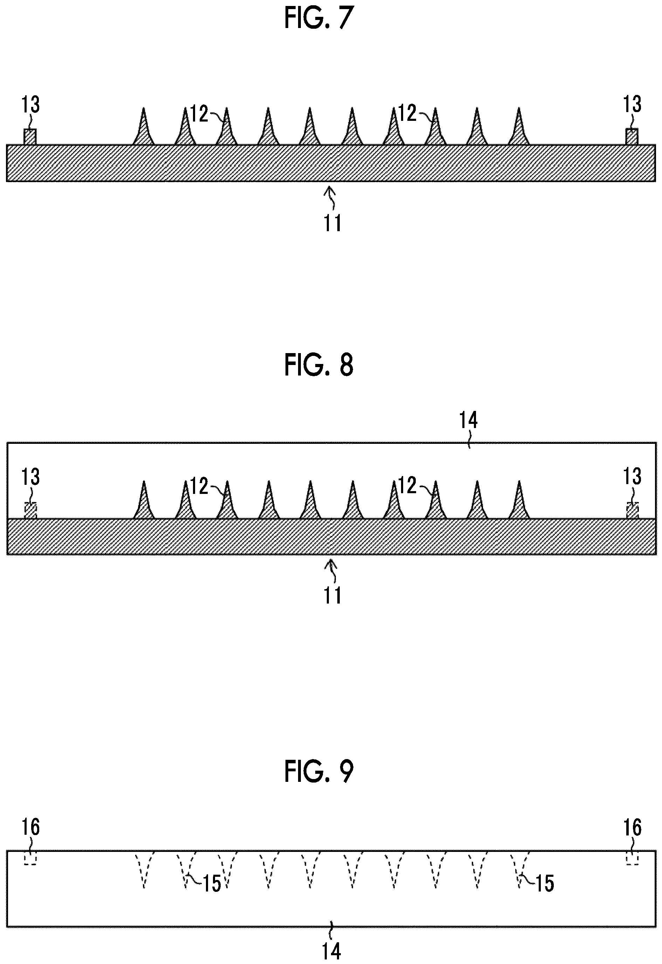

Figs. 7 to 9 are process diagrams of the production of a mold.

As illustrated in Fig. 7, a plate precursor for producing a mold for manufacturing a percutaneous absorption sheet is produced first.

There are two kinds of production methods of a plate precursor 11. In the first method, a photoresist is applied onto a Si substrate, and exposure and development are performed thereon. In addition, etching such as reactive ion etching (RIE) is performed, thereby producing an array of a plurality of protrusions 12 having the same shape as the needle-like protrusions of the percutaneous absorption sheet on the surface of the plate precursor 11. In addition, groove protrusions 13 having inverted shapes of grooves of the mold. Furthermore, in the case where etching such as RIE is performed to form the protrusions on the surface of the plate precursor 11, it is possible to form the protrusions 12 by performing etching in an oblique direction while rotating the Si substrate.

In the second method, there is a method of producing the plurality of protrusions 12 and the groove protrusions 13 on the surface of the plate precursor 11 through processing using a cutting tool such as a diamond tool on a metal substrate made of Ni or the like.

Next, as illustrated in Fig. 8, a mold 14 is produced using the plate precursor 11. For producing a typical mold 14, a method by Ni electroforming or the like is used. Since the plate precursor 11 has the protrusions 12 having a conical shape or pyramid shape (for example, square pyramid shape) with an acute-angled distal end, the shape is accurately transferred to the mold 14, and the mold 14 can be peeled away from the plate precursor 11. Furthermore, four methods that enable cheap manufacturing are considered.

The first method is a method in which a silicone resin obtained by adding a hardener to polydimethylsiloxane (PDMS, for example, SYLGARD 184 Dow Corning Corporation) is poured to a plate precursor 11 and is subjected to a heat treatment at 100°C to cure, and a mold 14 is peeled away from the plate precursor 11. The second method is a method in which a UV curable resin which is cured by being irradiated with UV radiation is poured to a plate precursor 11 and is irradiated with UV radiation in a nitrogen atmosphere, and a mold 14 is peeled away from the plate precursor 11. The third method is a method in which a solution obtained by dissolving a plastic resin such as polystyrene or polymethyl methacrylate (PMMA) in an organic solvent poured to a plate precursor 11 to which a release agent is applied and is dried so as to cause the organic solvent to be vaporized for curing, and a mold 14 is peeled away from the plate precursor 11. The fourth method is a method of preparing an inverted produce by Ni electroforming.

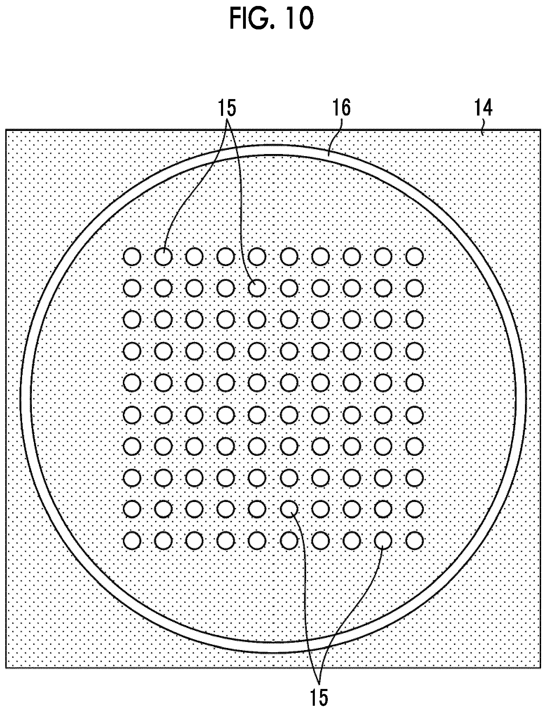

The mold 14 produced as described above is illustrated in Fig. 9, and the plan view of the mold 14 is illustrated in Fig. 10. By the method described above, the mold 14 in which needle-like recesses 15 having inverted shapes of the protrusions 12 of the plate precursor 11 are two-dimensionally arranged and annular grooves 16 are formed around the needle-like recesses 15 is produced. In any of the four methods described above, it is possible to easily and repeatedly produce the mold 14.

Although Figs. 7 to 9 illustrate the method in which the groove protrusions 13 having the inverted shapes of the grooves 16 are provided in the plate precursor 11 and the grooves 16 are provided by transfer, the manufacturing method of the grooves 16 is not limited thereto. After a mold 14 is produced using a plate precursor 11 having no groove protrusions 13, grooves 16 may be formed by cutting. However, in this case, a step of forming the grooves 16 in each mold is necessary. Therefore, it is preferable to form the mold 14 by providing the groove protrusions 13 in the plate precursor 11.

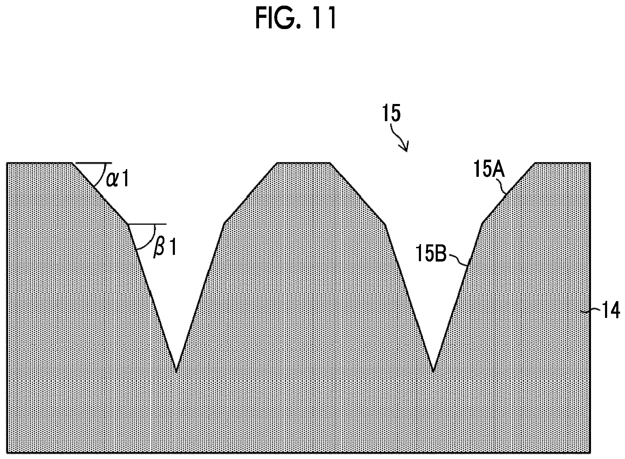

Fig. 11 is a partial enlarged view of the needle-like recesses 15 of the mold 14. The needle-like recess 15 is provided with a tapered inlet portion 15A narrowing in the depth direction from the surface of the mold 14 and a distal end recess 15B tapered in the depth direction. The taper angle α1 of the inlet portion 15A is basically identical to the angle α between the side surface of the frustum portion of the percutaneous absorption sheet and the sheet portion. The taper angle β1 of the distal end recess 15B is basically identical to the angle β between the side surface of the needle portion and the upper base of the frustum portion.

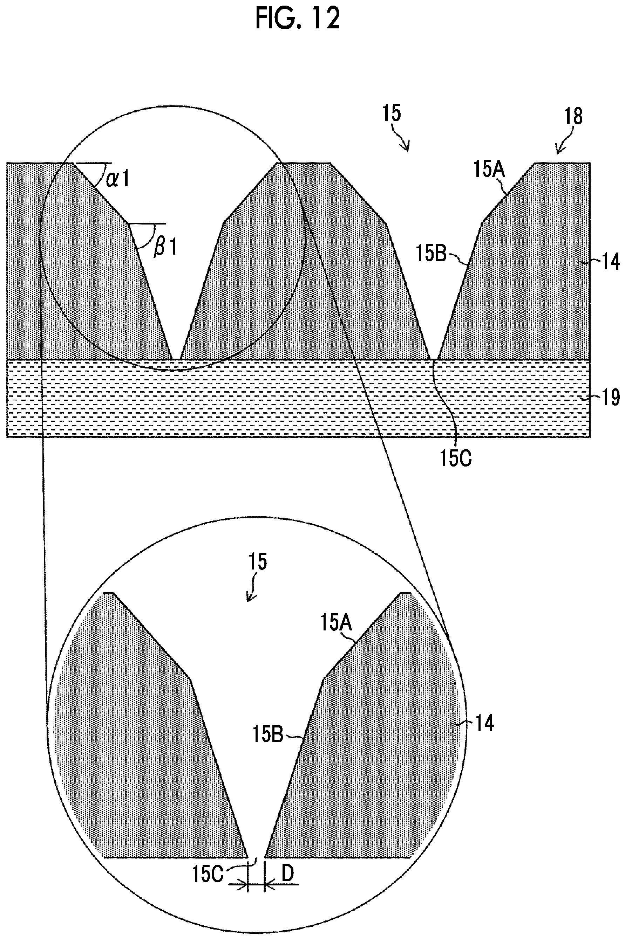

Fig. 12 illustrates an embodiment of a mold complex 18 that is more preferable for carrying out the manufacturing method of the percutaneous absorption sheet. As illustrated in Fig. 12, the mold complex 18 is constituted by a mold 14 in which a through-hole 15C is formed at the distal end of a needle-like recess 15 and a gas permeable sheet 19 which is bonded to the through-hole 15C side of the mold 14 and is formed of a material that allows gas to permeate therethrough but does not allow liquid to permeate therethrough. The distal end of the needle-like recess 15 communicates with the atmosphere via the gas permeable sheet 19 by the through-hole 15C. The distal end of the needle-like recess 15 means the side tapered in the depth direction of the mold 14 and means the side opposite to the side filled with a polymer solution.

By using the mold complex 18, a percutaneous absorption material solution filling the needle-like recess 15 is not permeated and only the air existing in the needle-like recess 15 can be released from the needle-like recess 15 through the through-hole 15C. The transferability achieved in a case where the shape of the needle-like recess 15 is transferred to the percutaneous absorption material is improved, and a sharper needle-like protrusion can be formed.

The diameter D of the through-hole 15C is preferably in a range of 1 to 50 µm. In this range, the air can be easily released, and the distal end portion of the needle-like protrusion of the percutaneous absorption sheet can be formed into a sharp shape. As the gas permeable sheet 19 which is formed of the material that allows gas to permeate therethrough but does not allow liquid to permeate therethrough, for example, POREFLON (trademark, Sumitomo Electric Industries, Ltd.) can be suitably used.

As the material used for the mold 14, a resin material or a metal material can be used. Among these, a resin material is preferable, and a material having high gas permeability is more preferable. The oxygen permeability, which is a representative of the gas permeability, is preferably higher than 1 × 10⁻¹² (mL/s·m·Pa), and more preferably higher than 1 × 10⁻¹⁰ (mL/s·m·Pa). By setting the gas permeability to be in the above range, the air existing in the needle-like recess 15 of the mold 14 can be released from the mold 14 side. A percutaneous absorption sheet with less defects can be manufactured. As such a material, as the resin material, general engineering plastics such as a silicone resin, an epoxy resin, polyethylene terephthalate (PET), polymethyl methacrylate (PMMA) polystyrene (PS), polyethylene (PE), polyacetal or polyoxymethylene (POM), polytetrafluoroethylene (PTFE), an ultraviolet (UV) curable resin, a phenol resin, and a urethane resin can be used. As the metal material, Ni, Cu, Cr, Mo, W, Ir, Tr, Fe, Co, MgO, Ti, Zr, Hf, V, Nb, Ta, α-aluminum oxide, stainless steel, and alloys thereof can be employed. Furthermore, as will be described later, since it is necessary to fix a second polymer solution in the grooves in a second polymer solution supplying step, a material of which the water repellency and wettability are controlled is preferably used for the mold 14. For example, it is preferable that the contact angle between the mold and the second polymer solution is greater than 90° or close to 90°.

### (Polymer Solution)

A polymer solution which is a solution of the polymer resin used in this embodiment will be described.

In this embodiment, a polymer solution which contains a predetermined amount of a drug and forms the drug layer 120 of the percutaneous absorption sheet 100 illustrated in Fig. 1 is referred to as a first polymer solution or a polymer solution containing a drug, and a polymer solution which forms the polymer layer 122 is referred to as a second polymer solution. In a case of being simply referred to as a polymer solution, the polymer solution indicates both the first polymer solution and the second polymer solution. Whether or not a predetermined amount of a drug is contained is determined based on whether or not a medicinal effect is exhibited as the body surface is punctured. Therefore, containing a predetermined amount of a drug means containing a drug in an amount that exhibits a medicinal effect as the body surface is punctured.

As a material of a resin polymer used for the polymer solution, it is preferable to use a biocompatible resin. As such resins, sugars such as glucose, maltose, pullulan, sodium chondroitin sulfate, sodium hyaluronate, and hydroxyethyl starch, proteins such as gelatin, and biodegradable polymers such as polylactic acid and a lactic acid-glycolic acid copolymer are preferably used. Among these, since gelatin-based materials have adhesiveness to many base materials and have a strong gel strength in a case of being used as a gelating material, the gelatin-based materials can be brought into close contact with a base material in a peeling step, which will be described later, and a polymer sheet can be peeled away from the mold using the base material. Therefore, the gelatin-based materials can be suitably used. Although the concentration varies depending on the material, it is preferable that the concentration is set so that 10 to 50 mass% of the resin polymer is contained in the second polymer solution. The solvent used for dissolution may not be warm water as long as the solvent is volatile, and methyl ethyl ketone, alcohol, or the like may be used. In addition, it is possible to dissolve the drug, which is supplied into the body according to the application, in the solution of the polymer resin. The polymer concentration of the first polymer solution (the concentration of the polymer excluding the drug in a case where the drug itself is a polymer) is preferably 0 to 30 mass%.

As a method of preparing the polymer solution, in a case where a water-soluble polymer (such as gelatin) is used, a water-soluble powder may be dissolved in water and the drug may be added after the dissolution. Otherwise, a powder of a water-soluble polymer may be dissolved in a liquid in which the drug is dissolved. In a case where it is difficult to dissolve the polymer in water, heating may be performed for dissolution. The temperature can be appropriately selected depending on the kind of the polymer material, and it is preferable that heating is performed at a temperature of about 60°C or lower. For the first polymer solution, the viscosity of the solution of the polymer resin is preferably 100 Pa·s or less, and more preferably 10 Pa·s or less. For the second polymer solution, the viscosity is preferably 2000 Pa·s or less, and more preferably 1000 Pa·s or less. By appropriately adjusting the viscosity of the solution of the polymer resin, easy injection of the solution into a needle-like recess of a mold is facilitated. For example, the viscosity of the solution of the polymer resin can be measured with a capillary viscometer, a falling ball viscometer, a rotational viscometer, or a vibrational viscometer.

### (Drug)

The drug to be contained in the polymer solution is not particularly limited as long as the drug has a function as a drug. Particularly, the drug is preferably selected from peptides, proteins, nucleic acids, polysaccharides, vaccines, pharmaceutical compounds belonging to water-soluble low molecular compounds, or cosmetic ingredients.

### (Surfactant)

A surfactant may be contained in the second polymer solution. By including a surfactant in the second polymer solution, the surface tension of the second polymer solution can be decreased. Specifically, it is preferable that the surface tension thereof is set to be lower than that of water (72.7 mN/m). The second polymer solution is pinned in the grooves 16 in the second polymer solution supplying step or a drying step, which will be described later. By decreasing the surface tension of the second polymer solution, the contractile force of the second polymer solution becomes weak such that the second polymer solution can be easily fixed in the grooves. In addition, since wettability to the mold is improved, the second polymer solution can be caused to smoothly fill the grooves, and the incorporation of bubbles (air) can be suppressed.

As the surfactant, any one of anionic, cationic, or nonionic surfactant can be used. However, from the viewpoint of not affecting the polymer solution, it is preferable to use a nonionic surfactant. For example, POLYSORBATE 20, 60, 80 or PLURONIC can be used. In a case where the concentration of the surfactant in the polymer solution is too low, the surface tension does not decrease. In a case where the concentration thereof is too high, the surfactant is not dissolved in water and forms precipitates insoluble after the application, so that a uniform surface is not achieved. Therefore, as in a case of a general coating liquid, it is preferable to set the concentration to be 0.01 vol% or more and 5 vol% or less.

### (Manufacturing Method of Percutaneous Absorption Sheet)

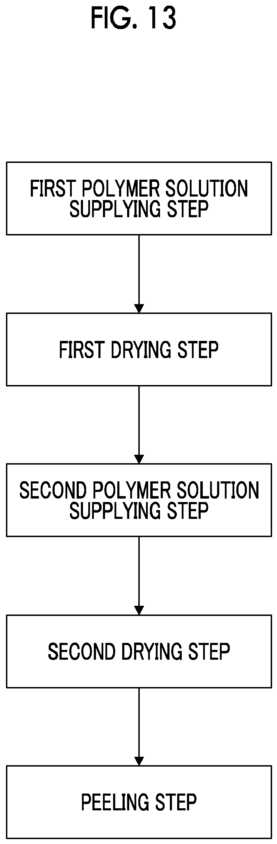

As an example of the manufacturing method of the sheet having needle-like protrusions, a manufacturing method of the percutaneous absorption sheet described above will be described. As illustrated in Fig. 13, the manufacturing method of the percutaneous absorption sheet includes a first polymer solution supplying step, a first drying step, a second polymer solution supplying step, a second drying step, and a peeling step. The first drying step may be omitted and the first polymer solution and the second polymer solution may be simultaneously dried as a drying step after the second polymer solution supplying step.

### (First Polymer Solution Step)

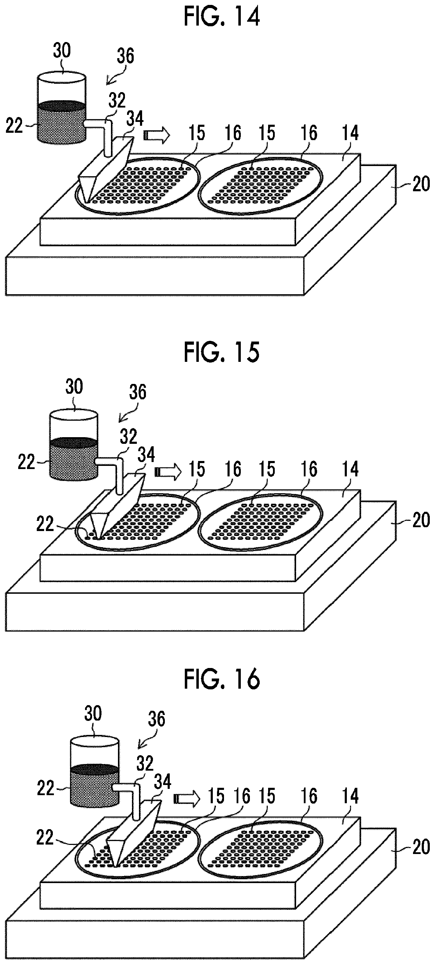

The manufacturing method of the percutaneous absorption sheet using the mold 14 will be described. As illustrated in Fig. 14, the mold 14 having the needle-like recesses 15 which are two-dimensionally arranged is disposed on a base 20. In the mold 14, two sets of 10 × 10 needle-like recesses 15 two-dimensionally arranged are formed. A liquid supply device (corresponding to "applicator") 36 having a liquid feed tank 30 which stores a first polymer solution 22, a pipe 32 connected to the liquid feed tank 30, and a nozzle 34 connected to the distal end of the pipe 32 is prepared. The first polymer solution 22 is discharged from the distal end of the nozzle 34.

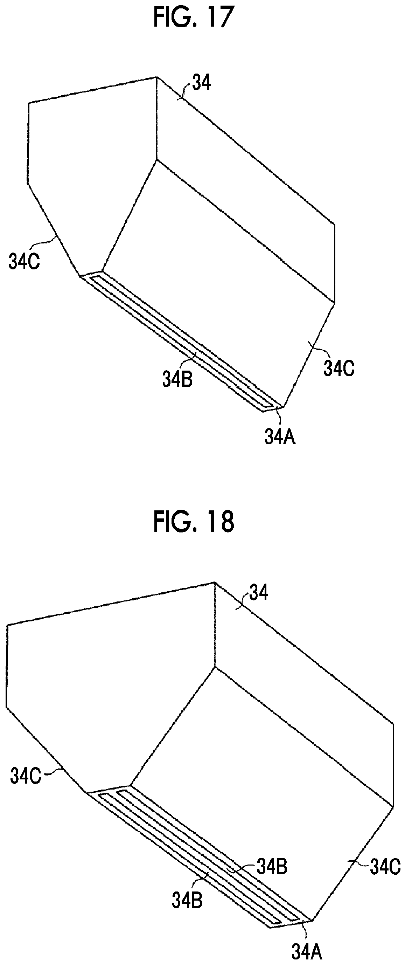

Fig. 17 is a schematic perspective view of the distal end portion of the nozzle. As illustrated in Fig. 17, the nozzle 34 has a lip 34A which is a flat surface on the distal end side, a slit-shaped opening 34B, two inclined surfaces 34C extending along the lip 34A in a direction away from the opening 34B. For example, it is possible to simultaneously fill a plurality of the needle-like recesses 15 constituting one row with the first polymer solution 22 by the slit-shaped opening 34B. The size (length and width) of the opening 34B is appropriately selected according to the number of needle-like recesses 15 to be filled at once.

By increasing the length of the opening 34B, a larger number of needle-like recesses 15 can be filled with the first polymer solution 22 at once. This makes it possible to improve productivity. Furthermore, it is preferable that the width of the opening 34B is set such that the opening 34B does not pass over the grooves 16 during the supply of the first polymer solution. In a case where the opening 34B is present on the grooves 16, there may be a case where the grooves 16 are filled with the first polymer solution. In a case where the width of the opening 34B is set to be large and the opening 34B passes over the grooves 16, by causing the corner formed by the wall surface of the groove 16 and the surface of the mold to be in a range of more than 90° and equal to or less than 135°, which will be described later, the time for which the opening 34B passes over the grooves 16 can be one second or shorter, thereby preventing the first polymer solution to be supplied to the grooves 16.

Fig. 18 illustrates a schematic perspective view of the distal end portion of another nozzle. As illustrated in Fig. 18, the nozzle 34 is provided with a lip 34A which is a flat surface on the distal end side, two slit-shaped openings 34B, and two inclined surfaces 34C extending along the lip 34A in a direction away from the openings 34B. For example, it is possible to simultaneously fill a plurality of the needle-like recesses 15 constituting two rows with the first polymer solution 22 by the two openings 34B.

As a material to be used for the nozzle 34, an elastic material or a metal material can be used. For example, TEFLON (registered trademark), stainless steel, and titanium can be employed.

Returning to Fig. 15, a filling step will be described with reference to Fig. 15. As illustrated in Fig. 15, the position of the opening 34B of the nozzle 34 is adjusted to be above the needle-like recesses 15. The nozzle 34 through which the first polymer solution 22 is discharged is pressed against the mold 14 and the lip 34A of the nozzle 34 and the surface of the mold 14 are brought into contact with each other. The first polymer solution 22 is supplied from the liquid supply device 36 to the mold 14 such that the needle-like recesses 15 are filled with the first polymer solution 22 from the opening 34B of the nozzle 34. In this embodiment, the plurality of needle-like recesses 15 constituting one row are simultaneously filled with the first polymer solution 22. However, the filling manner is not limited thereto, and the needle-like recesses 15 can be filled one by one. Alternatively, by using the nozzle 34 illustrated in Fig. 18, regarding a plurality of needle-like recesses 15 constituting a plurality of rows, every the plurality of rows can be simultaneously filled with the first polymer solution 22.

In a case the mold 14 is formed of a material having gas permeability, the first polymer solution 22 can be suctioned by being suctioned from the rear surface of the mold 14, and the filling of the needle-like recesses 15 with the first polymer solution 22 can be accelerated.

Subsequent to the filling step illustrated in Fig. 15, as illustrated in Fig. 16, the liquid supply device 36 is relatively scanned in a direction perpendicular to the longitudinal direction of the opening 34B while the lip 34A of the nozzle 34 and the surface of the mold 14 are brought into contact with each other. The nozzle 34 is scanned over the mold 14, and the nozzle 34 is moved to the needle-like recesses 15 which are not filled with the first polymer solution 22 yet. The position of the opening 34B of the nozzle 34 is adjusted to be above the needle-like recesses 15. In this embodiment, an example in which the nozzle 34 is scanned is described. However, the mold 14 may also be scanned.

Since the nozzle 34 is scanned over the mold 14 while the lip 34A of the nozzle 34 and the surface of the mold 14 are brought into contact with each other, the nozzle 34 can scrape off the first polymer solution 22 remaining on the surface of the mold 14 other than the needle-like recesses 15. The first polymer solution 22 can be caused not to remain on portions of the mold 14 other than the needle-like recesses 15. Furthermore, in this embodiment, the nozzle 34 is disposed such that the inclined surface 34C is at a position perpendicular to the scanning direction indicated by the arrow. Therefore, the nozzle 34 can be smoothly scanned over the mold 14.

In order to suppress deformation due to the compression of the mold 14 as much as possible by reducing damage to the mold 14, it is preferable to control the extent to which the nozzle 34 is pressed against the mold 14 at the time of scanning. For example, it is preferable to control the pressing force of the nozzle 34 against the mold 14 and the pressing distance of the nozzle 34 into the mold 14. In addition, it is desirable that at least one of the mold 14 or the nozzle 34 is made of a flexible material that is elastically deformable so as not to cause the first polymer solution 22 to remain in portions of the mold 14 other than the needle-like recesses 15.

By repeating the filling step of Fig. 15 and the scanning step of Fig. 16, the 10 × 10 needle-like recesses 15 that are two-dimensionally arranged are filled with the first polymer solution 22. In a case where the 10 × 10 needle-like recesses 15 that are two-dimensionally arranged are filled with the first polymer solution 22, the liquid supply device 36 is moved to the adjacent 10 × 10 needle-like recesses 15 that are two-dimensionally arranged, and the filling step of Fig. 15 and the scanning step of Fig. 16 are repeated. The adjacent 10 × 10 needle-like recesses 15 that are two-dimensionally arranged are also filled with the first polymer solution 22.

Regarding the filling step and the scanning step described above, (1) an embodiment in which the needle-like recesses 15 are filled with the first polymer solution 22 while scanning the nozzle 34 may be employed, or (2) an embodiment in which the nozzle 34 is temporarily stopped above the needle-like recesses 15 during scanning of the nozzle 34 to supply the first polymer solution 22 and the nozzle 34 is scanned again after the supply may be employed. During the filling step and the scanning step, the lip 34A of the nozzle 34 is pressed against the surface of the mold 14. It is preferable that the amount of the first polymer solution 22 discharged from the liquid supply device 36 is equal to the total volume of the plurality of needle-like recesses 15 of the mold 14 to be filled. By causing the first polymer solution 22 not be remain on portions of the mold 14 other than the needle-like recesses 15, the loss of the drug can be reduced.

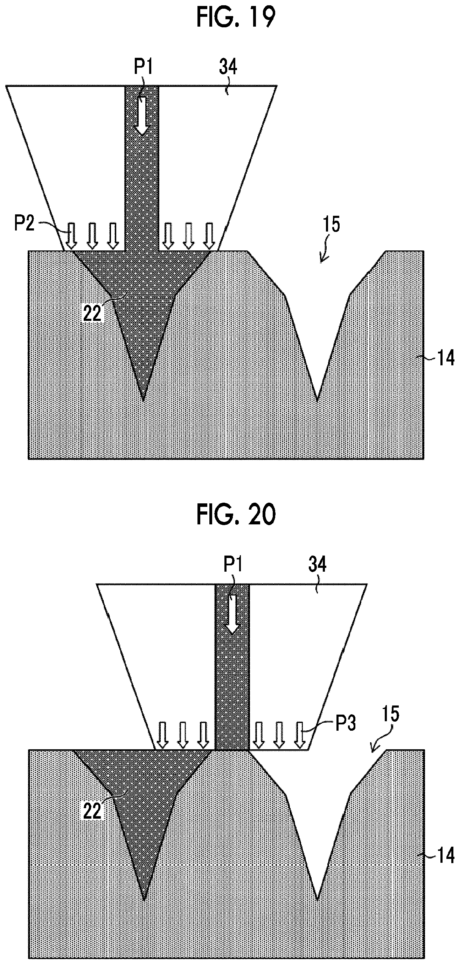

Fig. 19 is a partial enlarged view of the distal end of the nozzle 34 and the mold 14 while the needle-like recess 15 is filled with the first polymer solution 22. As illustrated in Fig. 19, by applying a pressurizing force P1 into the nozzle 34, filling of the needle-like recess 15 with the first polymer solution 22 can be accelerated. Furthermore, in a case where the needle-like recess 15 is filled with the first polymer solution 22, a pressing force P2 for bringing the nozzle 34 into contact with the surface of the mold 14 is preferably equal to or higher than the pressurizing force P1 in the nozzle 34. By achieving the pressing force P2 ≥ the pressurizing force PI, the leakage of the first polymer solution 22 from the needle-like recess 15 to the surface of the mold 14 can be suppressed.

Fig. 20 is a partial enlarged view of the distal end of the nozzle 34 and the mold 14 during the movement of the nozzle 34. In a case where the nozzle 34 is scanned relative to the mold 14, a pressing force P3 for bringing the nozzle 34 into contact with the surface of the mold 14 is preferably smaller than the pressing force P2 for bringing the nozzle 34 into contact with the surface of the mold 14 during the filling. This is to reduce damage to the mold 14 and suppress deformation due to compression of the mold 14.

The lip 34A of the nozzle 34 is preferably parallel to the surface of the mold 14. The posture of the nozzle 34 may be controlled by providing a joint drive mechanism at a portion to which the nozzle 34 is attached.

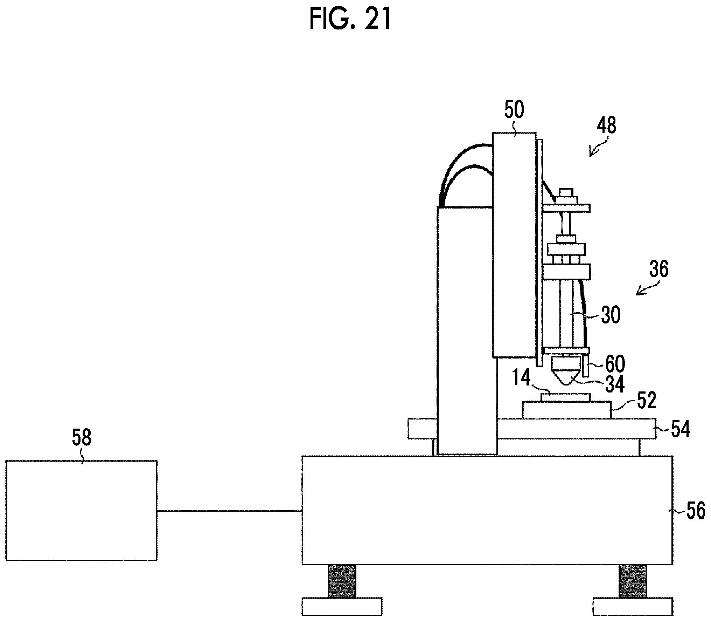

It is preferable to control the pressing force and/or the pressing distance of the nozzle 34 into the mold 14 by driving the nozzle 34 in a Z-axis direction according to the surface shape of the mold 14. Fig. 21 is a schematic configuration diagram of a polymer solution filling apparatus 48 that can control the pressing force and/or the pressing distance. The polymer solution filling apparatus 48 has the liquid supply device 36 having the liquid feed tank 30 which stores the first polymer solution and the nozzle 34 attached to the liquid feed tank 30, a Z-axis driving unit 50 which drives the liquid feed tank 30 and the nozzle 34 in the Z-axis direction, a suction base 52 on which the mold 14 is placed, an X-axis driving unit 54 which drives the suction base 52 in an X-axis direction, a table 56 which supports the apparatus, and a control system 58.

A case where the pressing force is controlled to be constant will be described. The nozzle 34 is brought close to the mold 14 by the Z-axis driving unit 50 up to a Z coordinate at which a desired pressing force is achieved. While the nozzle 34 in contact with the mold 14 is scanned by the X-axis driving unit 54, the first polymer solution 22 is discharged while controlling the Z-axis coordinate so as to cause the pressing force to become constant. A contact pressure measurement method is not particularly limited. For example, various load cells can be used, for example, under the suction base 52 or instead of the suction base 52. The load cell means a measuring instrument capable of measuring the compressive force in a thickness direction. The pressing force is preferably controllable to be constant at arbitrary pressure in a range of 1 to 1000 kPa against the mold 14.

A case where the pressing distance is controlled to be constant will be described. The surface shape of the mold 14 is measured in advance before being brought into contact with the nozzle 34. While the nozzle 34 in contact with the mold 14 is being scanned by the X-axis driving unit 54, the first polymer solution 22 is discharged while feeding a value obtained by offsetting a Z-axis coordinate to achieve a desired pressing distance into the surface shape of the mold 14 back to the Z-axis driving unit 50.

The shape measurement method is not particularly limited. For example, an optical measuring instrument such as a non-contact type laser displacement meter 60, a contact type stylus profilometer, or the like can be used. Furthermore, the posture of the nozzle 34 in the slit direction may be controlled according to the surface shape of the mold 14. The pressing distance is preferably controlled to be in a range of 1% to 15% with respect to the thickness of the mold 14. By performing an operation while controlling the distance between the nozzle 34 and the mold 14 according to the shape of the mold 14 in the Z-axis direction by the Z-axis driving unit 50, the compressive deformation ratio is uniformized and the filling amount accuracy can be improved.

Regarding the control of the pressing force and the pressing distance, in a case where the pressing distance is short, it is preferable to control the pressing force, and in a case where the pressing distance is long, it is preferable to directly control the pressing distance.

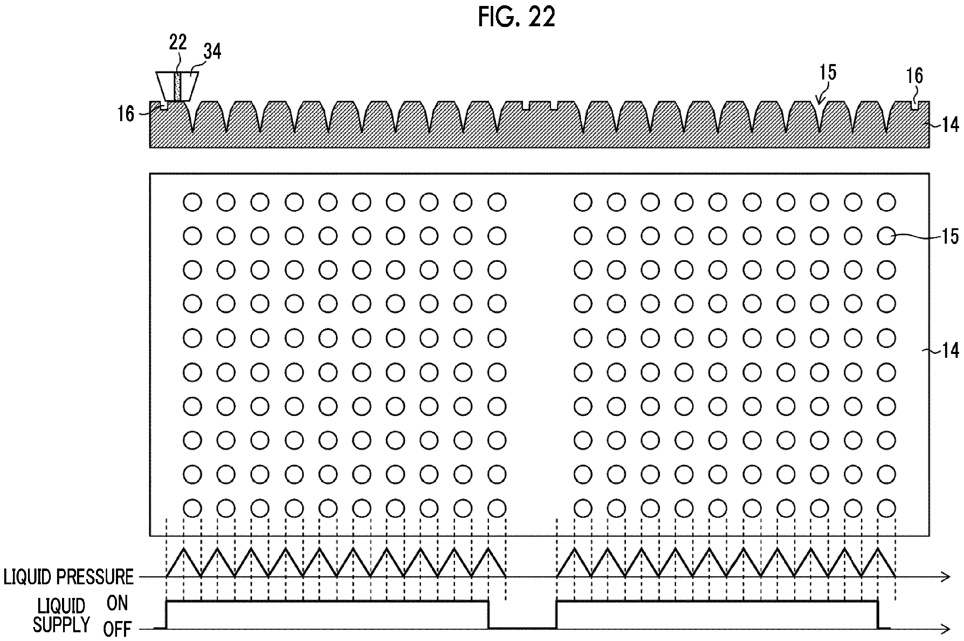

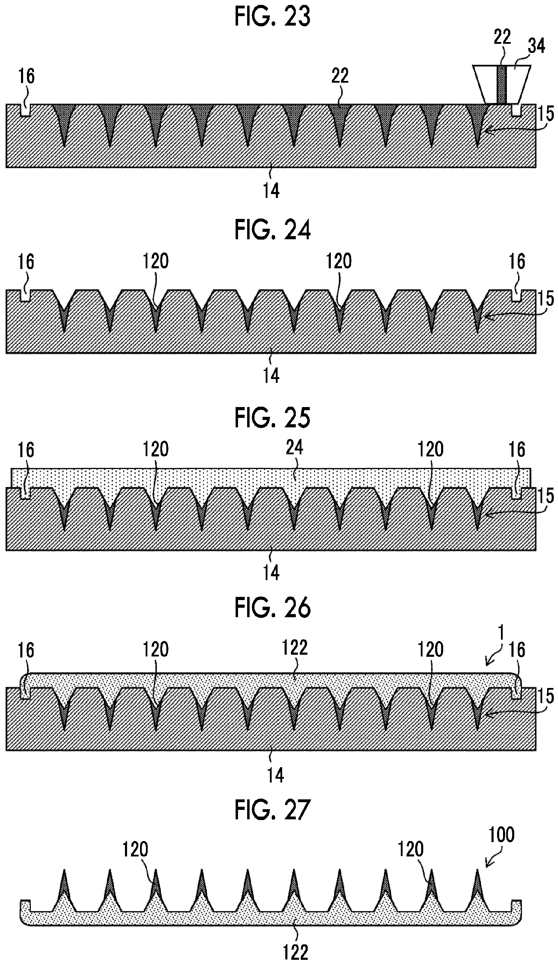

Fig. 22 is an explanatory view illustrating the relationship between the liquid pressure in the nozzle and the supply of the first polymer solution. As illustrated in Fig. 24, the supply of the first polymer solution 22 is started before the nozzle 34 is positioned above the needle-like recesses 15. This is to reliably fill the needle-like recesses 15 with the first polymer solution 22. The first polymer solution 22 is continuously supplied to the mold 14 until the filling of the plurality of 10 × 10 needle-like recesses 15 is completed. The supply of the first polymer solution 22 to the mold 14 is stopped before the nozzle 34 is positioned above the needle-like recesses 15 in the tenth row. The first polymer solution 22 can be prevented from overflowing from the needle-like recesses 15. Regarding the liquid pressure in the nozzle 34, in a case where the supply of the first polymer solution 22 is started, the nozzle 34 is increased in height in a region where the needle-like recesses 15 are not positioned. On the other hand, in a case where the nozzle 34 is positioned above the needle-like recesses 15, the needle-like recesses 15 are filled with the first polymer solution 22 and the liquid pressure in the nozzle 34 decreases. The fluctuation of the liquid pressure is repeated.

In a case where the filling of the plurality of 10 × 10 needle-like recesses 15 is completed, the nozzle 34 is moved to the plurality of 10 × 10 needle-like recesses 15 adjacent thereto. Regarding the supply of the liquid, it is preferable to stop supplying the first polymer solution 22 in the case where the nozzle 34 is moved to the plurality of 10 × 10 needle-like recesses 15 adjacent thereto. There is a distance from the needle-like recesses 15 in the tenth row to the needle-like recesses 15 in the next row. In a case where the first polymer solution 22 is continuously supplied while the nozzle 34 is being scanned therebetween, there may be a case where the liquid pressure in the nozzle 34 becomes too high. As a result, there is a case where the first polymer solution 22 flows out from the nozzle 34 to portions of the mold 14 other than the needle-like recesses 15, and in order to suppress this, it is preferable to stop supplying the first polymer solution 22.

In a case of filling with the first polymer solution 22, it is preferable to use the nozzle 34 after cleaning the distal end of the nozzle 34. This is because in a case where there is a deposit on the surface of the lip 34A of the nozzle 34 before filling, the accuracy of the filling amount of the first polymer solution 22 decreases. For cleaning, wiping with a nonwoven fabric is generally used. At the time of wiping, in a case where the nonwoven fabric is impregnated with water, a solvent, or the like, cleaning can be effectively performed.

There is a possibility that in a case where the nozzle 34 is separated from the mold 14 after the filling with the first polymer solution 22, the first polymer solution 22 may remain on the surface of the mold 14. By performing suck-back control to suction the first polymer solution 22 from the opening 34B of the nozzle 34 after the completion of the filling of the needle-like recesses 15, an excessive amount of the first polymer solution 22 discharged is suctioned and the remnant of the liquid on the surface of the mold 14 can be reduced.

In the first polymer solution supplying step, by using the mold 14 illustrated in Fig. 12, the first polymer solution 22 can be suctioned from the through-hole 15C side to fill the needle-like recess 15.

In a case where the filling of the needle-like recesses 15 with the first polymer solution 22 is completed, the process proceeds to the first drying step, the second polymer solution supplying step, the second drying step, and the peeling step.

As illustrated in Fig. 23, in the first polymer solution supplying step, the needle-like recesses 15 of the mold 14 are filled with the first polymer solution 22 from the nozzle 34. The first polymer solution supplying step is performed in the above-described method.

### (First Drying Step)

As illustrated in Fig. 24, in the first drying step, the first polymer solution 22 is dried to solidify, thereby forming the drug layer 120, which is to become a first layer, in the needle-like recesses 15.

The first drying step is a step of drying the first polymer solution 22 filling the needle-like recesses 15 of the mold 14 to cause the first polymer solution 22 to be localized at the distal ends of the needle-like recesses 15. The first drying step is preferably performed in an environment with a temperature of 1°C or higher and 10°C or lower. By performing the first drying step in this range, the occurrence of bubble defects can be reduced. In addition, by optimizing the drying rate by controlling the temperature and humidity conditions of the first drying step, sticking of the first polymer solution 22 to the wall surface of the mold 14 in the needle-like recess 15 can be reduced, and the drying proceeds while the first polymer solution 22 is gathered at the distal end of the needle-like recess 15 by the drying.

It is preferable that the drying of the first polymer solution 22 in the first drying step is performed in a windless state. In a case where uneven wind directly hits the first polymer solution 22, drying unevenness occurs. This is because the drying rate of the portion strongly hit by the wind increases, sticking of the first polymer solution 22 to the wall surface of the mold 14 occurs, and there is a possibility that the first polymer solution 22 may be prevented from being localized at the distal end of the needle-like recess 15.

In order to realize drying in a windless state, it is preferable to install a draft shield, for example. The draft shield is installed so that the mold 14 is not directly hit by the wind. As the draft shield, a method of installing a physical obstacle such as a lid, an awning, a screen, or a fence is simple and preferable. In the case where the draft shield is installed, it is preferable to secure a vent and the like so as not to cause the installation space of the mold 14 to be in an enclosed state. In the enclosed state, there is a possibility that the water vapor in the enclosed space may be saturated and the drying of the first polymer solution 22 may not proceed. It is preferable that the vent allows vapor to flow in and out, and in order to stabilize the air flow inside the draft shield, it is more preferable to cover the vent with a water vapor permeable film or the like. The drying time is appropriately adjusted in consideration of the shape of the needle-like recess 15, the arrangement and the number of needle-like recesses 15, the kind of the drug, the filling amount and the concentration of the first polymer solution 22, and the like.

The windless state refers to a case where the wind speed is 0.5 m/s or less as well as a state where there is no wind at all. This is because drying unevenness rarely occurs in this range.

In the first drying step, the first polymer solution 22 is dried to solidify such that the first polymer solution 22 shrinks to a smaller size than the filling state. Accordingly, it is possible to easily peel the drug layer 120 away from the needle-like recesses 15 of the mold 14 in the peeling step.

### (Second Polymer Solution Supplying Step)

Next, as illustrated in Fig. 25, the second polymer solution 24 is supplied onto the surface of the mold 14 above the drug layer 120 containing a predetermined amount of the drug to fill the needle-like recesses 15 and the grooves 16 with the second polymer solution 24. Since the drug layer 120 is solidified by the drying, the diffusion of the drug contained in the drug layer 120 into the second polymer solution 24 can be suppressed. For the supply of the second polymer solution 24, dispenser coating, bar coating, spin coating, spray coating, or the like can be applied, but the application is not limited thereto. In a case where the mold 14 is formed of a gas permeable material, by supplying the second polymer solution 24 onto the mold 14 to close the needle-like recesses 15 and the grooves 16 with the second polymer solution and thereafter suctioning the second polymer solution from the rear surface side of the mold 14, the needle-like recesses 15 and the grooves 16 can be filled with the second polymer solution and the air in the needle-like recesses 15 and the grooves 16 can be released.

In the second polymer solution supplying step, the second polymer solution 24 is supplied to a wider range than the grooves 16 formed in the mold 14 as seen from the upper surface of the mold 14. The second polymer solution 24 is repelled on the surface of the mold 14 and contracts due to surface tension. In a case where the liquid applied to the outside of the grooves 16 reaches the grooves 16 due to the contraction of the second polymer solution 24, the second polymer solution 24 is fixed in the grooves 16. By performing the second drying step as the subsequent step in the fixed state, the shape of the polymer layer 122 (the shape of the sheet portion 116) can be stably formed. In addition, polymer sheets can be individually manufactured without the connection of adjacent polymer sheets. Therefore, the manufacturing of sheets can be performed without performing a cutting step.

In addition, even though the second polymer solution 24 is simply supplied and the second polymer solution 24 does not contract to reach the grooves 16 due to the contraction on the mold 14, the second polymer solution 24 can be dried to contract in the second drying step as the subsequent step and thus can be fixed in the grooves 16. Therefore, the shape of the polymer layer 122 (the shape of the sheet portion 116) can be stably formed.

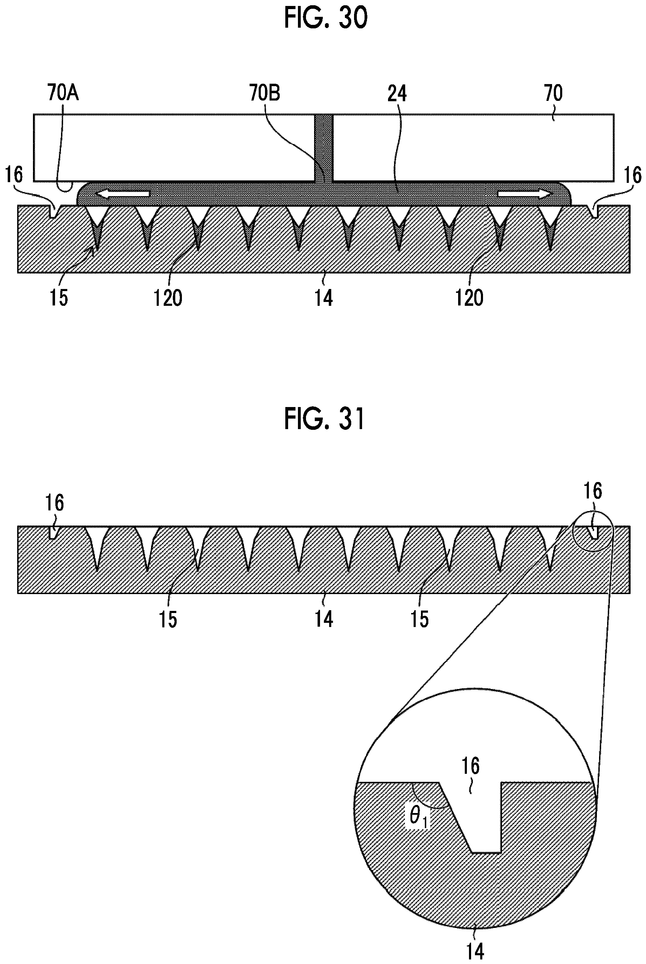

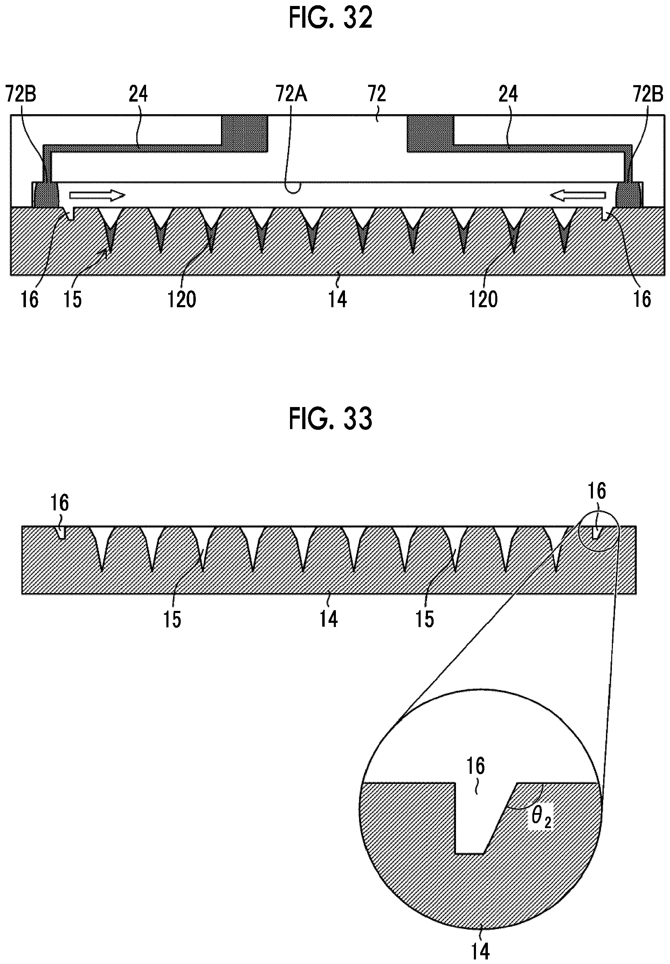

The width of the groove 16 formed in the mold 14 is preferably 2 mm or less. By causing the width of the groove 16 to be 2 mm or less, the amount of the filling second polymer solution can be reduced. In addition, in the first polymer solution supplying step, filling of the grooves 16 with the first polymer solution can be prevented. Furthermore, it is preferable that the width of the groove 16 is 20 µm or more. By causing the width to be 20 µm or more, the grooves 16 can be filled with the second polymer solution, and fixing of the second polymer solution can be reliably performed. The width of the groove 16 refers to the shortest distance from one end to the other end on the surface of the mold 14, and in a case of a groove illustrated in Figs. 31 and 33 in which the wall surface is inclined, which will be described later, refers to the distance from the position where the inclination of the wall surface starts.

The height of the groove 16 is preferably lower than the height of the needle-like recess 15. By causing the height of the groove 16 to be lower than the height of the needle-like recess 15, in a case where the formed percutaneous absorption sheet is pierced, a protrusion having an inverted shape of the groove 16 can be prevented from becoming an obstacle. In a case where the height of the groove 16 is low, the air in the groove 16 can be easily removed during filling with the second polymer solution. In the second polymer solution supplying step, since the air in the groove 16 is easily released, the incorporation of bubbles in the groove 16 can be prevented during fixing of the second polymer solution. In a case where bubbles are incorporated, the fixed second polymer solution tends to deviate from the position with the incorporated bubbles. Furthermore, by causing the height of the groove 16 to be high, the contact area of the second polymer solution in the groove 16 can be increased, and thus the deviation of the fixed second polymer solution can be prevented. Therefore, it is preferable that the height of the groove 16 is determined by the condition of the second polymer solution filling the groove 16 and the condition of the air released from the groove 16.

### (Second Drying Step)

After the second polymer solution supplying step, as illustrated in Fig. 26, the second polymer solution 24 is dried to solidify, thereby forming the polymer layer 122, which is to become a second layer, on the drug layer 120. A polymer sheet 1 which is a laminate of the drug layer 120 and the polymer layer 122 is manufactured.

In the second drying step, the volume of the second polymer solution 24 is reduced by the drying. In a case where the second polymer solution 24 is fixed in the groove 16 of the mold 14 during the drying, a reduction in the volume occurs in the film thickness direction of the sheet, resulting in a reduction in the film thickness.

### (Peeling Step)

A method of peeling the polymer sheet 1 away from the mold 14 is not limited. It is desirable that the needle-like protrusion is not bent or folded during the peeling. Specifically, after a sheet-like substrate on which a pressure sensitive adhesive layer with pressure sensitive adhesive properties is formed adheres onto the polymer sheet 1, the peeling can be formed by bending the substrate back from the end portion thereof. Alternatively, a method in which a sucker is installed on the rear surface of the polymer sheet 1 and the rear surface is pulled up vertically while being suctioned with air can be applied. By peeling the polymer sheet 1 away from the mold 14, a percutaneous absorption sheet 100 is manufactured (Fig. 27).

### (Degassing Step)

It is preferable to degas the first polymer solution 22 and the second polymer solution 24 before supplying the first polymer solution 22 and the second polymer solution 24 to the mold 14. By the degassing, bubbles contained in the first polymer solution 22 and the second polymer solution 24 can be removed before filling of the needle-like recesses 15 of the mold 14. For example, in the degassing step, bubbles having a diameter of 100 µm to several mm are removed.

Examples of the degassing method include (1) a method of exposing the first polymer solution 22 to a reduced pressure environment for 1 to 15 minutes, (2) a method in which a container storing the first polymer solution 22 is subjected to ultrasonic vibration for 5 to 10 minutes, (3) a method of applying ultrasonic waves to the first polymer solution 22 while being exposed to a reduced pressure environment, and (4) a method in which helium gas is introduced into the first polymer solution 22 to replace dissolved gas with helium. The degassing methods (1) to (4) can also be applied to the second polymer solution 24.

### [Another Embodiment of Mold]

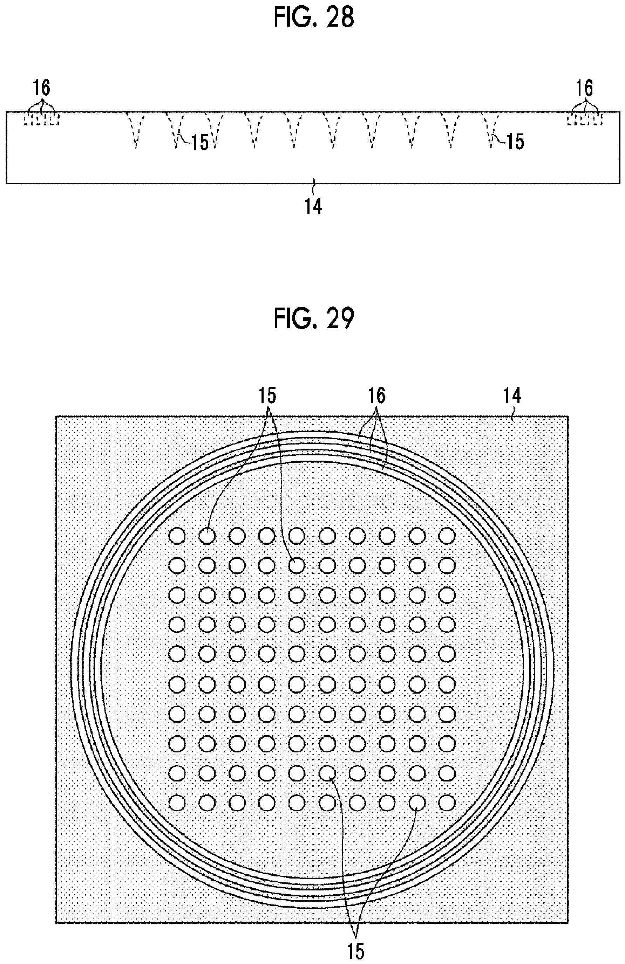

Figs. 28 and 29 are views illustrating a mold of another embodiment, in which Fig. 28 is a sectional view, and Fig. 29 is a plan view. A mold 14 illustrated in Figs. 28 and 29 has three concentric grooves 16 having different radii. By providing a plurality of the grooves 16, even in a case where fixing of the second polymer solution in the outermost groove cannot be performed, fixing in the next inner groove can be performed. Therefore, the contraction of the second polymer solution can be stopped at the grooves, and thus a percutaneous absorption sheet can be manufactured within a predetermined size standard.

### [Another Embodiment of Second Polymer Solution Supplying Step]

Another embodiment of the second polymer solution supplying step will be described with reference to Figs. 30 and 31. In the second polymer solution supplying step (illustrated in Fig. 25), a dispenser is used to supply the second polymer solution from above the needle-like recesses 15 and the grooves 16. However, Fig. 30 is different in that the second polymer solution is supplied onto a region where the needle-like recesses 15 are formed to fill the grooves 16 while moving the second polymer solution on the surface of the mold 14.

As illustrated in Fig. 30, a dispenser 70 used in this embodiment has a lip 70A and an opening 70B at the distal end of the dispenser 70. The opening 70B is provided at the center of the distal end of the dispenser 70 and discharges the second polymer solution 24. The lip 70A is a flat surface, and the second polymer solution 24 discharged from the opening 70B fills the grooves 16 while moving between the lip 70A and the surface of the mold 14. The size of the lip 70A is preferably in a range larger than the groove 16 in the case where the dispenser 70 is disposed on the mold 14 so that the second polymer solution 24 can be guided to the groove 16.

Since the distance between the surface of the mold 14 and the lip 70A of the dispenser 70 becomes the thickness of the coating film of the second polymer solution 24, the distal end of the dispenser 70 is preferably brought close to the surface of the mold 14 during the supply of the second polymer solution 24.

Fig. 31 is a sectional view illustrating the shape of a mold 14 which is preferably used in the second polymer solution supplying step illustrated in Fig. 30. The mold 14 illustrated in Fig. 31 is different from the other molds in that the shape of a groove 16 is formed so that the wall surface on a region side where needle-like recesses 15 are formed. Assuming that the wall surface of the region side where the needle-like recesses 15 are formed is an inner wall surface and the wall surface on the opposite side thereof is an outer wall surface, the shape of the groove 16 illustrated in Fig. 31 is preferably formed so that the corner formed by the inner wall surface and the mold surface is at an obtuse angle, and specifically, the angle θ₁ is preferably more than 90° and equal to or less than 135°. In this embodiment, since filling of the grooves 16 with the second polymer solution 24 is performed while moving the surface of the mold 14 from the region side where the needle-like recesses 15 are formed, by causing the angle of the inner wall surface to be in the above range, the second polymer solution can be easily introduced into the grooves 16. By filling the grooves 16 with the second polymer solution 24, the second polymer solution can be brought into contact with the side surface and the bottom surface of the grooves 16, and thus pinning of the second polymer solution 24 can be reliably performed.

In addition, the angle of the corner formed by the outer wall surface and the mold surface is preferably smaller than the angle (θ₁) of the corner formed by the inner wall surface and the mold surface, and is more preferably a right angle (90°). By causing the angle of the outer wall surface to be in the above range, the second polymer solution 24 can be easily pinned in the grooves 16 during contraction. When the angle is caused to be smaller than 90°, the distal end of the protrusion having an inverted shape of the groove 16 is large and becomes an obstacle in a case where the formed polymer sheet is peeled. Therefore, by causing the angle to be 90° or more, peeling of the polymer sheet can be easily performed.

Figs. 32 and 33 are views illustrating another embodiment of the second polymer solution supplying step. In the second polymer solution supplying step illustrated in Fig. 32, the second polymer solution 24 is supplied from around the groove 16.

As illustrated in Fig. 32, a dispenser 72 used in this embodiment has a lip 72A and an opening 72B at the distal end of the dispenser 72. It is preferable that the position of the opening 72B is on the outside of the groove 16 in the case where the dispenser 72 is disposed on the mold 14 so that the second polymer solution 24 is discharged from the opening 72B and the second polymer solution 24 is supplied around the groove 16 of the mold 14.

The lip 72A is a flat surface, and the second polymer solution 24 discharged from the opening 72B fills the grooves 16 and the needle-like recesses 15 while moving between the lip 72A and the surface of the mold 14. Since the distance between the surface of the mold 14 and the lip 72A of the dispenser 72 becomes the thickness of the coating film of the second polymer solution 24, the distal end of the dispenser 72 is preferably brought close to the surface of the mold 14 during the supply of the second polymer solution 24.

Fig. 33 is a sectional view illustrating the shape of a mold 14 which is preferably used in the second polymer solution supplying step illustrated in Fig. 32. The mold illustrated in Fig. 33 is different from the other molds in that the shape of a groove 16 is formed so that the wall surface on the opposite side to the wall surface on a region side where needle-like recesses 15 are formed, that is, the wall surface on the side where the second polymer solution 24 is supplied is inclined. The shape of the groove 16 illustrated in Fig. 32 is preferably formed so that the corner formed by the outer wall surface and the mold surface is at an obtuse angle, and specifically, the angle θ₂ is preferably more than 90° and equal to or less than 135°. By causing the corner formed by the outer wall surface and the mold surface to be at an angle in the above range, the second polymer solution can be easily introduced into the groove 16 and can be reliably pinned.

The angle of the corner formed by the inner wall surface and the mold surface is preferably smaller than the angle (θ₂) of the corner formed by the outer wall surface and the mold surface, and is more preferably vertical (90°). By causing the angle of the inner wall surface to be in the above range, the second polymer solution 24 can be easily pinned in the grooves 16 during contraction. When the angle is caused to be smaller than 90°, the distal end of the protrusion having an inverted shape of the groove 16 is large and becomes an obstacle in a case where the formed polymer sheet is peeled. Therefore, by causing the angle to be 90° or more, peeling of the polymer sheet can be easily performed.

In the description of the mold illustrated in Figs. 31 and 33, the number of grooves is one, but a plurality of grooves 16 may be provided. In the case of providing a plurality of grooves, it is preferable that the wall surfaces of all the grooves on the side where the second polymer solution is supplied are inclined.

### [Examples]

Hereinafter, the present invention will be described more specifically with reference to examples of the present invention. The materials, use amount, proportion, processing content, processing procedure, and the like described in the following examples can be appropriately changed without departing from the gist of the present invention. Therefore, the scope of the present invention should not be interpreted restrictively by the following specific examples.

### <<Experimental Example 1>>

### (Production of Mold)

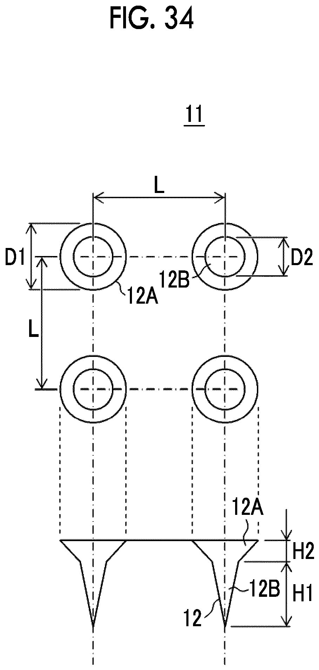

Protrusions 12 having a needle-like structure in which a cone 12B having a diameter D2 of 300 µm and a height H1 of 500 µm is formed on a truncated cone 12A having a bottom surface diameter D1 of 500 µm and a height H2 of 150 µm as illustrated in Fig. 34 were ground in a two-dimensional array of 10 rows and 10 columns at a pitch L of 1000 µm on the surface of a smooth Ni plate with a side length of 40 mm. Furthermore, on the outer periphery thereof, a groove protrusion which was centered in the middle of the two-dimensional array of 10 rows and 10 columns and had a radius of 9.5 mm from the center, a protrusion width of 0.1 mm (100 µm), and a height of 250 µm was ground, thereby producing a plate precursor 11. In addition, a plate precursor 11 having a radius of 9.5 mm from the center and a groove protrusion width of 0.5 to 3 mm was produced. Furthermore, circular protruding plate precursors 11 having a radius of 6 mm and 12 mm from the center and a groove protrusion width of 1 mm were produced such that a plurality of different kinds of plate precursor 11 were prepared. The inverted shape of the groove protrusion became a groove of a mold. As a comparative example, a plate precursor without a groove protrusion was also produced.

A film of a silicone rubber (SILASTIC-MDX4-4210 manufactured by Dow Corning Corporation) was formed on each of the plate precursors 11 into a thickness of 0.6 mm, was thermally cured, and was peeled off. The silicone rubber inverted product was trimmed down to a flat surface portion with a side length of 30 mm in which 10 rows and 10 columns of needle-like recesses two-dimensionally arranged were formed at the center portion and was used as a mold. A side where the opening of the needle-like recess was side was set to the surface of the mold and the opposite side was set to the rear surface of the mold.

### (Preparation of First Polymer Solution)

Hydroxyethyl starch (manufactured by Fresenius Kabi) was dissolved in water to prepare an 8% aqueous solution, and 2 mass% of human serum albumin (manufactured by Wako Pure Chemical Industries, Ltd.) was added thereto to prepare a simulated drug solution. After the preparation, the resultant was exposed to a reduced pressure environment at 3 kPa for 4 minutes for sufficient degassing.

### (Preparation of Second Polymer Solution)

Chondroitin sulfate (manufactured by Maruha Nichiro Corporation) was dissolved in water to prepare a 40% aqueous solution, and this was used as a polymer layer forming liquid. After the preparation, the resultant was exposed to a reduced pressure environment at 3 kPa for 4 minutes for sufficient degassing.

Hereinafter, the entire manufacturing steps of a sheet having needle-like protrusions were performed under an environment at a temperature of 5° and a relative humidity of 35 %RH.

### (First Polymer Solution Supplying Step and Drying Step)

A first polymer solution filling apparatus includes a driving unit for controlling relative position coordinates of a mold and a nozzle in X and Z axes, a liquid supply device (super small amount fixed-quantity dispenser SMP-III manufactured by Musashi Engineering, Inc.) to which the nozzle can be attached. a suction base for fixing the mold, a laser displacement meter (HL-C201A manufactured by Panasonic Corporation) for measuring the surface shape of the mold, a load cell (LCX-A-500N manufactured by Kyowa Electronic Instruments Co., Ltd.) for measuring the nozzle pressing pressure, and a control system for controlling the Z axis based on the data of the measurement values of the surface shape and the pressing pressure.

The mold was installed on the horizontal suction base so that the surface thereof faced upward. A suction pressure at a gauge pressure of -90 kPa was applied in a direction from the rear surface of the mold to fix the mold to the suction base.

A nozzle made of stainless steel (SUS) having a shape as illustrated in Fig. 17 was prepared, and a slit-like opening having a length of 12 mm and a width of 0.2 mm was formed at the center of a lip having a length of 20 mm and a width of 2 mm. The opening was sized to fit in a region where 10 rows and 10 columns of two-dimensionally arranged needle-like recesses formed on the mold were formed. By causing the size of the opening to be this size, the first polymer solution could be supplied only to the needle-like recesses. The nozzle was connected to the tank. 3 mL of the first polymer solution was loaded in the tank and the nozzle. The nozzle was adjusted so that the opening was parallel to the first row constituted by a plurality of the needle-like recesses formed in the surface of the mold. The nozzle was pressed against the mold at a pressure (pressing force) of 0.14 kgf/cm² (1.4 N/cm²) at a position spaced 2 mm away from the first row in the opposite direction to the second row. While pressing the nozzle and while moving the nozzle in a direction perpendicular to the longitudinal direction of the opening at 1 mm/sec while controlling the Z axis to cause the variation in the pressing force to be within ±0.05 kgf/cm² (0.49 N/cm²), the first polymer solution was discharged from the opening by the liquid supply device at 0.31 µL/sec for 10 seconds. The movement of the nozzle was stopped at a position spaced 2 mm away from the tenth row of the plurality of needle-like recesses two-dimensionally arranged in the opposite direction to the ninth row, and the nozzle was separated from the mold. The mold filled with the first polymer solution was placed in a draft shield (25 cm³) having an opening with a diameter of 5 mm and was dried. The draft shield mentioned here had a structure in which a gas permeable film (POREFLON FP-010 manufactured by Sumitomo Electric Industries, Ltd.) was attached to the opening such that the wind is not directly applied.

### (Second Polymer Solution Supplying Step and Drying Step)

The second polymer solution was spotted on the mold filled with the first polymer solution by a dispenser while adjusting the discharge amount and the clearance between the mold and the nozzle. 60 mg of the second polymer solution was spotted in a range wider than the groove of the mold by 1 mm in the radial direction. Thereafter, it was checked whether or not the shape of the percutaneous absorption sheet was maintained after 12 hours had elapsed. Evaluation was performed according to the following standard. Thereafter, the second polymer solution was dried and peeled off. The results are shown in Table 1.

### <<Liquid Surface Fixing>>

A...The polymer layer was fixed in the groove of the mold.
B...The polymer layer was not fixed in the groove of the mold, the second polymer solution was repelled on the mold, and the shape of the percutaneous absorption sheet was not stable.

**[Table 1]**

| Test No. | Radius of groove [mm] | Width of groove [mm] | Evaluation (liquid surface fixing) | Example / Comparative Example |
|---|---|---|---|---|
| 1 | None | - | B | Comparative Example |
| 2 | 9.5 | 3 | A | Example |
| 3 | 9.5 | 2 | A | Example |
| 4 | 9.5 | 1 | A | Example |
| 5 | 9.5 | 0.5 | A | Example |
| 6 | 9.5 | 0.1 | A | Example |
| 7 | 6 | 1 | A | Example |
| 8 | 12 | 1 | A | Example |

In Test Nos. 2 to 8 in which molds provided with grooves were used, the second polymer solution was fixed in the grooves. Contrary to this, in Test No 1 in which a mold with no grooves were used, the second polymer solution was repelled and contracted on the mold and sheets with a constant size could not be formed. In addition, in Test Nos. 2 to 8, the second polymer solution could be fixed in any of the examples. However, in Test No 2 in which the width of the groove was 3 mm, the first polymer solution was introduced into the grooves during filling with the first polymer solution. In Test Nos. 3 to 8 in which the width of the groove was 2 mm or less, the sheet could be formed without the first polymer solution filling the grooves or being caught in the grooves.

### <<Experimental Example 2>>

The number of grooves formed on the outer periphery of the needle-like recesses was increased concentrically to the mold of Test No 5 of Experimental Example 1. Specifically, grooves having a groove width of 0.5 mm were formed adjacent to each other with an interval of 0.1 mm in the radial direction from a groove having a radius of 9.5 mm and a groove width of 0.5 mm, and a mold was produced by using a plate precursor in which triple concentric circles were produced. Molding experiments were conducted on the produced mold in the same manner as in Example 1.

As a result, in one molding experiment, no difference was found in the effect between a single groove and triple grooves. However, in the result of molding of sheets repeated 30 times, while the liquid surface fixing of the single groove was evaluated as "A" for 25 times and evaluated as "B" for 5 times, the liquid surface fixing of the three grooves was evaluated as "A" for all the 30 times. From the viewpoint of stability, by forming multiple grooves, the second polymer solution is caught in the grooves, and even in a case where the second polymer solution deviates from one groove, the second polymer solution is caught in the next adjacent groove. Therefore, the sheet can be stably manufactured.

### <<Experimental Example 3>>

Experiments were conducted by changing the shape of the groove for a mold having a groove with a radius of 9.5 mm, a groove width of 0.5 mm and a groove height of 0.25 mm (corresponding to Test No. 5 in Experimental Example 1). Evaluation of the shape of the groove was conducted by using a mold in which, regarding an inner wall surface on the region side where the needle-like recesses of the grooves were formed, the angle of the corner formed by the wall surface and the mold surface was 120° and regarding an outer wall surface on the opposite side, the angle of the corner formed by the wall surface of the mold surface was 90°, a mold in which, regarding an outer wall surface on the opposite side to the region side where the needle-like recesses of the grooves were formed, the angle of the corner formed by the wall surface and the mold surface was 120° and regarding an inner wall surface on the region side where the needle-like recesses of the grooves were formed, the angle of the corner formed by the wall surface of the mold surface was 90°, and a mold (the mold used in Experimental Examples 1 and 2) in which, regarding an inner wall surface and an outer wall surface, the corner formed by the wall surface and the mold surface was at 90°. In addition, as a comparative example, the experiments were also conducted on a mold with no grooves.

Regarding the supply of the second polymer solution, the second polymer solution was supplied in a method (Fig. 30) of supplying the second polymer solution to the grooves from the region side where the needle-like recesses were formed by using the filling head illustrated in Figs. 30 and 32 as the filling head of a dispenser, and a method (Fig. 32) of supplying the second polymer solution from around the grooves. Sheets were molded by each of the supplying methods, and fixing of the liquid surface in the grooves and entrainment of bubbles into the grooves were visually checked. The results are shown in Table 2.

**[Table 2]**

| Test No. | Angle of inner wall surface in groove [°] | Angle of outer wall surface in groove [°] | Liquid supplying method | Inclusion of bubbles [times / 30 times] | Failure in liquid surface fixing [times / 30 times] | Example / Comparative Example |
|---|---|---|---|---|---|---|
| 11 | No groove | | Supply from inside | - | 30 | Comparative Example |
| 12 | 90 | 90 | Supply from inside | 5 | 7 | Example |
| 13 | 90 | 90 | Supply from outside | 7 | 9 | Example |
| 14 | 120 | 90 | Supply from inside | 0 | 0 | Example |
| 15 | 120 | 90 | Supply from outside | 5 | 6 | Example |
| 16 | 90 | 120 | Supply from inside | 3 | 3 | Example |
| 17 | 90 | 120 | Supply from outside | 0 | 0 | Example |

In Test No. 11 with no grooves, the liquid surface on the mold surface could not be fixed, and sheets with a constant size could not be molded. In Test Nos. 12 and 13 in which the inner wall surface and the outer wall surface of the groove were at 90°, entrainment of bubbles into the grooves was observed regardless of the second polymer solution supplying method. In a case where bubbles are entrained, there is concern that the peripheral portion of a part where bubbles are entrained may drop out during the peeling of the sheet after drying and dust may be caused in the steps. Furthermore, there is concern that the second polymer solution may repel the mold with the bubbles and may not be fixed in the grooves.

On the other hand, in Test Nos. 14 and 17 in which the angle of the wall surface on the supply side of the second polymer solution was 120°, entrainment of bubbles into the grooves did not occur. It is assumed that by causing the angle of the wall surface on the supply side to be 120°, the grooves could be smoothly filled during the supply of the second polymer solution such that incorporation of bubbles was eliminated.

### <<Example 4>>

Using the mold of Experimental Example 1 (radius 9.5 mm, groove width 0.5 mm), vacuum suction from the rear surface of the mold was performed during drying of the second polymer solution. In Table 2 and Test No. 12 of Experimental Example 3, the inclusion of bubbles had occurred five times out of 30 times. However, the incorporation of bubbles was eliminated by the vacuum suction from the rear surface side of the mold. It is considered that this is because the inside of the grooves is decompressed via the silicone rubber mold by the vacuum suction from the rear surface side and bubbles in the grooves are removed by diffusion.

### <<Example 5>>

Using the mold of Test No 11 of Experimental Example 3, sheets were formed by adding 0.1% of tween 80 (manufactured by Tokyo Chemical Industry Co., Ltd.) as a surfactant to the second polymer solution. In Test No. 11 of Experimental Example 3, while the inclusion of bubbles had occurred five times out of 30 times and the failure in liquid surface fixing had occurred seven times out of 30 times, by adding the surfactant, the inclusion of bubbles and the failure in liquid surface fixing could be reduced to 0 out of 30 times.

It is considered that this is because the addition of the surfactant caused a reduction in the surface tension of the second polymer solution, a reduction in the contractive force, an improvement of the wettability to the silicone mold, and a reduction in the contact angle such that the grooves could be smoothly filled with the second polymer solution during the filling and the incorporation of bubbles was eliminated.

### Explanation of References

1: polymer sheet
11: plate precursor
12: protrusion
12A: cone
12B: truncated cone
13: groove protrusion
14: mold
15: needle-like recess
15A: inlet portion
15B: distal end recess
15C: through-hole
16: groove
18: mold complex
19: gas permeable sheet
20: base
22: first polymer solution
30: liquid feed tank
32: pipe
34: nozzle
34A, 70A, 72A: lip
34B: opening
34C: inclined surface
36: liquid supply device
48: polymer solution filling apparatus
50: Z-axis driving unit
52: suction base
54: X-axis driving unit
56: table
58: control system
60: displacement meter
70, 72: dispenser
100: percutaneous absorption sheet
110: needle-like protrusion
112: needle portion
112A: needle-like portion
112B: body portion
114: frustum portion
116: sheet portion
120: drug layer
122: polymer layer

## Claims

1. A manufacturing method of a sheet having needle-like protrusions comprising:
for a mold provided with needle-like recesses and an annular groove provided around a region where the needle-like recesses are formed,
a first polymer solution supplying step of supplying a first polymer solution, which is to become a first layer, to the needle-like recesses;
a second polymer solution supplying step of filling the needle-like recesses and the groove with a second polymer solution by supplying the second polymer solution, which is to become a second layer, to a surface of the mold;
a drying step of forming a laminate of the first layer and the second layer by drying the first polymer solution and the second polymer solution; and
a peeling step of peeling the laminate away from the mold.

2. The manufacturing method of a sheet having needle-like protrusions according to claim 1,
wherein, after the second polymer solution supplying step, the second polymer solution is pinned in the groove.

3. The manufacturing method of a sheet having needle-like protrusions according to claim 1 or 2,
wherein a width of the groove is 2 mm or less.

4. The manufacturing method of a sheet having needle-like protrusions according to any one of claims 1 to 3,
wherein the groove consists of a plurality of annular grooves and further has one or more annular grooves around a single annular groove.

5. The manufacturing method of a sheet having needle-like protrusions according to claim 4, wherein the plurality of annular grooves are concentric grooves having different radii.

6. The manufacturing method of a sheet having needle-like protrusions according to any one of claims 1 to 5,
wherein an angle of a corner formed by a wall surface of the groove on a side of a region where the needle-like recesses are formed and the surface of the mold is an obtuse angle, and
in the second polymer solution supplying step, the second polymer solution is supplied from the needle-like recesses side to fill the groove.

7. The manufacturing method of a sheet having needle-like protrusions according to any one of claims 1 to 5,
wherein an angle of a corner formed by a wall surface of the groove on the opposite side to a region where the needle-like recesses are formed and the surface of the mold is an obtuse angle, and
in the second polymer solution supplying step, the second polymer solution is supplied from around the groove to fill the needle-like recesses and the groove.

8. The manufacturing method of a sheet having needle-like protrusions according to claim 6 or 7,
wherein the obtuse angle is an angle of more than 90° and equal to or less than 135°.

9. The manufacturing method of a sheet having needle-like protrusions according to any one of claims 6 to 8,
wherein an angle of a corner formed by a wall surface of the groove on the opposite side to a side where the second polymer solution is supplied and the surface of the mold is smaller than an angle of a corner formed by a wall surface on the side where the second polymer solution is supplied and the surface of the mold.

10. The manufacturing method of a sheet having needle-like protrusions according to claim 9,
wherein the angle of the corner formed by the wall surface of the groove on the opposite side to the side where the second polymer solution is supplied and the surface of the mold is a right angle.

11. The manufacturing method of a sheet having needle-like protrusions according to any one of claims 1 to 5,
wherein the mold is formed of a gas permeable material, and
in the second polymer solution supplying step, the groove is closed with the second polymer solution and thereafter the second polymer solution is suctioned from a side opposite to a surface of the mold in which the needle-like recesses and the groove are formed.

12. The manufacturing method of a sheet having needle-like protrusions according to any one of claims 1 to 11,
wherein the first polymer solution supplying step is performed while bringing a distal end of an applicator that applies the first polymer solution into contact with the mold.

13. The manufacturing method of a sheet having needle-like protrusions according to any one of claims 1 to 12,
wherein a surface tension of the second polymer solution is low.

14. The manufacturing method of a sheet having needle-like protrusions according to any one of claims 1 to 13,
wherein the first polymer solution includes a drug.
